(19) Europäisches Patentamt
European Patent Office
Office européen des brevets

(11) EP 1 920 250 B1

(12) EUROPEAN PATENT SPECIFICATION

(45) Date of publication and mention of the grant of the patent:
**23.02.2011 Bulletin 2011/08**

(21) Application number: **05782632.3**

(22) Date of filing: **31.08.2005**

(51) Int Cl.:
*G01N 33/52* (2006.01) *G01N 33/543* (2006.01)
*G01N 33/487* (2006.01) *B01L 3/00* (2006.01)
*G01N 21/00* (2006.01) *G02F 1/00* (2006.01)

(86) International application number:
**PCT/EP2005/009381**

(87) International publication number:
**WO 2007/025558 (08.03.2007 Gazette 2007/10)**

# (54) ANALYTE TEST SYSTEM USING NON-ENZYMATIC ANALYTE RECOGNITION ELEMENTS

ANALYTENTESTSYSTEM UNTER VERWENDUNG NICHTENZYMATISCHER ANALYTENERKENNUNGSELEMENTE

SYSTÈME DE TEST D'UN ANALYTE UTILISANT DES ÉLÉMENTS NON ENZYMATIQUES DE RECONNAISSANCE D'UN ANALYTE

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**14.05.2008 Bulletin 2008/20**

(73) Proprietor: **Egomedical Technologies AG**
**9242 Oberuzwil (CH)**

(72) Inventors:
- **STIENE, Matthias**
  **82205 Gilching (DE)**
- **HORSTKOTTE, Elke**
  **81245 München (DE)**
- **KUHLMEIER, Dirk**
  **80637 München (DE)**
- **JONES, Eurig, Wyn**
  **81377 München (DE)**

(74) Representative: **Graf von Stosch, Andreas et al**
**Graf von Stosch**
**Patentanwaltsgesellschaft mbH**
**Prinzregentenstrasse 22**
**80538 München (DE)**

(56) References cited:

| | |
|---|---|
| **EP-A- 0 503 914** | **EP-A- 1 574 858** |
| **EP-A1- 1 318 397** | **WO-A-02/076878** |
| **WO-A-03/083469** | **WO-A-2005/072216** |
| **WO-A1-2006/015615** | **WO-A2-02/085185** |
| **US-A- 4 761 381** | **US-A- 5 144 139** |
| **US-A- 6 165 739** | **US-A1- 2002 130 042** |
| **US-A1- 2003 083 685** | **US-A1- 2004 241 451** |
| **US-A1- 2005 196 820** | |

- **HEISS C ET AL: "DIP-AND READ TEST STRIPS FOR THE DETERMINATION OF TRINITROLTOLUENE (TNT) IN DRINKING WATER" ANALYTICA CHIMICA ACTA, ELSEVIER, AMSTERDAM, NL, vol. 396, no. 2/3, 20 September 1999 (1999-09-20), pages 309-316, XP001152739 ISSN: 0003-2670**
- **DOSCH M ET AL: "HOMOGENEOUS IMMUNOASSAY FOR THE DETECTION OF TRINITROTOLUENE (TNT) BASED ON THE REACTIVATION OF APOGLUCOSE OXIDASE USING A NOVEL FAD-TRINITROTOLUENE CONJUGATE" FRESENIUS JOURNAL OF ANALYTICAL CHEMISTRY, SPRINGER, BERLIN, DE, vol. 361, no. 2, May 1998 (1998-05), pages 174-178, XP000946964 ISSN: 0937-0633**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 920 250 B1

## Description

### Field of the Invention

The invention relates to an analyte test system for determining qualitatively and quantitatively an analyte in a liquid medium, such as an aqueous or physiological sample fluid. In a preferred embodiment the present invention relates to an analyte test system to qualify and quantify the reaction between a host and a guest molecule, particularly to affinity assays and immunoassays.

### Background of the invention

The diagnosis and confirmation of various illnesses and health status has been a major task for health care professionals since medicine has been practised. Currently, health physicians have at their disposal a multitude of diagnostic techniques available to them which allow the accurate diagnosis and confirmation of various diseases and patient conditions in bodily fluids. The determination of different patient conditions in particular diseases are of paramount importance to health practitioners, whereas different needs and requirements have to be met. The determination of different analytes in bodily fluids such as blood, serum, and urine, plays a significant role in the management of various diseases.

Physicians and patients require additionally an accurate test result systems and products which are easy to use and convenient, especially for home monitoring and point of care. Such convenience is strongly connected to the number of steps the physician or the patient has to perform to get the final test results, and to the required sample volume in case where the test system requires blood or serum as sample fluid and an invasive sampling procedure.

Typical examples are the detection of human Chorionic Gonadotrophin (hCG) in pregnancy tests or the determination of Cardiac Troponin I, Cardiac Troponin T, CKMB, Myoglobin, and BNP as cardiac markers to determine the time of the actual heart infarction.

Further examples for increasingly important analytes are tumour markers, which are useful and important to monitor treated patients to describe progress of the disease before any further cancerous tissue can be found clinically or by imaging. Examples of these markers are CEA, PSA, CA19-9, CA125. CEA, or carcinoembryonic antigen, a blood-borne protein, first noted to be produced by tumours of the gastrointestinal system.

Similarly, a further example of an increasingly important aspect of disease management and/or control being synonymous with complications relating to diabetes mellitus is diabetes retinopathy. Such a condition is a disease of the retina, and widely reported to occur in approximately a third of diabetic patients.

PSA, or Prostate Specific Antigen is produced by the normal prostate gland. It is an enzyme called a serine protease that usually acts as an anticoagulant to keep semen liquid. Only small amounts leak into the blood circulation in normal circumstances. Enlarged prostates and cancerous prostates leak substantial amounts in to the blood circulation and the urine, which can be detected for diagnosis and screening methods.

All the above tests and many others employ affinity reactions between the analyte of interest (guest molecule) and an analyte specific recognition element (host molecule) to qualify the existence or absence respectively to quantify the concentration of the analyte molecule.

One skilled in the art would classify the performed assay as immunoassay. In the case the affinity reaction as described in the above examples is carried out between an antibody or antibody fragment as a recognition element, the analyte of interest is then typically called an antigen. This type of affinity assay is most known and widely used in the field of clinical and medical diagnostics.

In 2003 9.6 million citizens were recorded to be living with cancer in the United States. Research by the American Cancer Society indicates that early detection and screening could reduce cancer deaths e.g. colorectal cancer by 30%, cervical cancer by 60%. Furthermore, other types of diseases which are on the increase in the western world include obesity, diabetes, and arthritis to name just a few. Furthermore, the diagnosis of diseases using immunoassays provide physicians with the opportunity to implement early medications or treatments to reduce the likelihood of having to perform complicated and more often than not, invasive treatments. Such treatments can be hazardous due to bacterial risk, expensive, and the likelihood of a patient making a full recovery is reduced per unit time after a disease has manifested itself within a patient's body.

In particular, whilst certain viruses are on the decrease throughout the world, others are on the increase and the World Health Organization has reported that by the year 2005 the estimated number of people affected by the Human Immunodeficiency virus (HIV) globally will be 40 million - an increase of 8% since the year 2000. Supplemental data suggests that third world or developing countries have noted a significant increase in the number of diagnosed individuals with such a disease and for some countries an increase of 15% over comparative dates is not uncommon.

A current problem therefore for physicians is their ability to provide a rapid and accurate test result to determine if a patient is suffering from a disease. One protocol for testing of diseases such as HIV, tuberculosis, cancer is to take

a blood sample from the patient and send it to a laboratory for analysis. Indeed, such a protocol can be problematic for a patient since the time delay between the initial consultation and receiving of a test result can cause mutual stress and unnecessary delay of the treatment and therefore increased risk for patients. However, point of care and 'walk-in' clinics are becoming prevalent in some countries to counteract the perceived inefficiency of Health Departments but the costs involved make them only attractive to the most affluent members of society.

In addition, test equipment at a physician's office may be expensive for some practices and some might prefer to rely on sending fluid samples to a laboratory for analysis. Additionally, the cost of seeking medical treatment in some countries by visiting a physician may be prohibitively expensive for some individuals since not all health schemes are free at the PoC. Furthermore, the diagnosis and monitoring of a disease can in some cases be a two fold exercise i.e., physicians require a confirmation whether a disease exists or not in a bodily fluid, and secondly, how developed is the disease by analysing the concentration level of an analyte in a bodily fluid.

Thus, individuals who may feel that they are at risk of an illness would benefit from a point of care test (PoC) or from a home diagnostic test whereas the latter system allows testing at their own comfortable surroundings and convenience. In addition, physicians would gain efficiency in their day to day business activities if they could perform a test diagnosis on site, without incurring expensive laboratory costs. In many cases, the supply of such diagnostic kits would give the patient an element of control of his illness.

Various tests elements do exist for either home testing of physiological conditions such as for diabetes, pregnancy, and fertility but there exists minimal test equipment that can provide a simple diagnostic tool to provide information of a disease state and progress with a fast and accurate result at a point of care or at home.

Different detection principles useful for laboratory test equipment and/or therapeutic test elements exist, which determine the presence and the level of an analyte in a test sample, e.g. Agglutination Immunoassay, Diffusion Immunoassay, Enzyme Modulator assays, Fluorescence Assisted Immunoassays, and Cofactor-Apo-enzyme-Assays.

Many modem industries and in particular the metabolic monitoring industry are therefore presented with the challenge of providing a test element with the following characteristics and properties:

- The test element should provide either accurate qualitative or accurate quantitative test results in aqueous and bodily fluids with as few handling steps for the user as possible.
- The test element should require only a small sample amount thus the physician or patient can use minimal invasive sampling methods.
- The test element should involve few production steps and therefore inexpensive manufacturing and should be usable for products assisting patients in self testing of physiological parameters and/or usable in a physician's place of work.

Prior art describes systems and examples to solve or partly solve the above quoted challenges, whereas the following cited publications are of particular interest to the disclosed invention.

United States Patent 4,213,893 discloses a Flavin Adenine Dinucleotide labelled conjugate being useful as labelled conjugate in specific binding assays for determining a ligand or a specific binding partner in liquid media such as serum. The FAD labelled conjugates used in binding assays for a ligand are monitored for the purposes of the assay by measuring FAD activity i.e. the co-enzyme or prosthetic group activity of the labelled conjugate.

United States Patent 4,708,933 discloses a homogeneous solid-state immunoliposome assay which utilizes the lateral phase separation of an antigenic liposome resulting in the destabilization and lysis of the liposome which may be quantified and employed in determining the presence and/or concentration of antigens, antibodies and like agents in biological fluids.

Clinical Chemistry 27: 1499-1504; Adaptation of Prostatic-Group-Label Homogeneous Immunoassay to reagent- strip format, published in 1981 to RJ Tyhach et aL, discloses a Prostatic-Group-Label Immunoassay (PGLIA) technique incorporated into a re-agent-strip format, using flavin N6- (N'-2,4-dinitrophenyl-6-aminohexyl)adenine dinucleotide (DNP-FAD) as the prosthetic group derivative and 6-N-(2,4- dinitrophenyl)aminohexanoic acid (DNP-caproate) as the competing ligand. DNP-FAD not bound by an antibody combines with glucose oxidase apo-enzyme, which reacts with glucose and oxygen, producing a colour through a peroxidase-linked system.

The rate of colour generation is thus a function of the DNP-caproate concentration. The Journal of Clinical Investigation 115:86-93 (2005); 'SDF-1 is both necessary and sufficient to promote proliferative retinopathy' published in 2005 to Butler et aL, discloses the link between a protein known as SDF-1 and retinopathy.

European Patent Application 163 393 discloses a latex immunoassay method to determine the presence of a ligand of interest in a non-extracted body fluid and includes a chemical additive comprised of a halogen substituted carboxylic acid or a salt of the acid in the reaction mixture to decrease non-specific interferences of the latex immunoassay. The addition of such additive permits the use of agglutination immunoassays to determine quantitative levels of endogenous metabolites and to monitor specific ligands of interest such as drugs, therapeutic agents and specific binding proteins in the samples.

WO2002/086472 discloses the use of fluorescent molecular rotors which vary in fluorescence intensity based

on viscosity of the environment The molecular rotors are modified with a hydrocarbon chain or hydrophilic group to allow for the measurement of membrane or liquid viscosity.

WO 2005/098431 discloses an analyte test system for the photometric detection and quantitative determination of an analyte, e.g. glucose, in a physiological fluid, e.g. blood, which is provided with an integrated calibration system using the standard addition method. The element comprises first and second surfaces forming a sample distribution system, the detection areas of which being coated with catalytic and calibration formulations respectively, which enable the detection/determination of an analyte by an enzymatic reaction.

However, up to now no analyte test system exists suitable for point of care or home settings, which can evaluate the non-enzymatic reaction between an analyte of interest and an analyte specific recognition element while performing an integrated calibration and/or quality control measurement for the analyte in question in a given physiological or aqueous sample fluid.

European Patent Application Publication 1 574 858 A1 is an earlier application by the present proprietor describing related subject-matter. It was published after the filing date of the present application. The same holds for WO 2006/015615. Both prior are documents disclose analyte test elements detecting the analyte upon a chemical reaction.

United States Patent 6,165,739 describes a stationary testing media device formed with a glass microscope slide divided into parallel capillary channels by strips of double-sided adhesive tape then covered with a thin cover glass.

International Patent Application Publication WO 02/076878 describes a method and structure for microfluidic flow guiding, which employs wettable and non-wettable surfaces to form a flow-path that has low flow resistance, promotes laminar flow and provides a superior gas-liquid interface.

## Summary of the Invention

The present invention provides an analyte test element as defined in claim 1, and a method for preparing an analyte test element as defined in claim 16. Preferred features of the invention are recited in the dependent claims.

Thus, the present invention provides an analyte test element for determining the concentration of at least one analyte in a physiological or aqueous sample fluid having a first surface and a second surface in a predetermined distance opposite from each other, said both surfaces are provided with two substantially equivalent patterns forming areas of high and low surface energy which are aligned mostly congruent, whereby the areas of high surface energy create a sample distribution system with at least two detection areas, wherein at least one of the detection areas of the first and second surfaces is provided with at least one specific non-enzymatic analyte affinity recognition element.

In a preferred embodiment, the analyte test element provides

*n* predetermined detection areas (6a) of the first surface (2a) coated with *n* calibration formulations (18) made up of *m* blank formulations and *n-m* formulations containing different levels of a calibration compound (33), whereby *n* is an integer number larger than 2, *m* is an integer number equal or larger than 1, and $n > m$, and

*n* predetermined detection areas (6'a) of the second surface (4a) coated with a formulation containing the specific non-enzymatic analyte affinity recognition element (32).

In a further aspect, the invention provides an analytes test system consisting of a meter device and an analyte test element, e.g. in shape of a strip, for non-enzymatic affinity reactions between an analyte of interest and a non-enzymatic analyte specific recognition element. For the integrated calibration and quality control mechanisms said analyte test system employs the standard addition methods utilising the sample distribution system of the analyte test element containing the required standards and/or quality controls. The system is optimised to perform immunoassays, receptor-assays, or other affinity assays in a swift and easy manner with a simple and disposable test element favourable for point of care and home testing.

The production of the inventive analyte test element involves only a small number of uncomplicated production steps enabling an inexpensive production of the element.

A better understanding of the features and advantages of the present invention will be obtained by reference to the following detailed description of illustrative and preferred embodiments in conjunction with the attached drawings.

## Brief Description of the Drawings

Figure 1 is a perspective view of one embodiment of the analyte test element of the present invention provided in a shape of a test strip.

Figure 2 is a perspective view of the embodiment according to Figure 1 showing a sample application area and the sample distribution system enlarged.

Figure 3 is an exploded perspective view of the element according to Figure 1 showing three separate construction layers.

Figure 4 shows different forms of a discontinuity of a centre layer forming a sample cavity together with the first and second layer.

Figure 5 is a sectional view of a detection area of the sample distribution system constructed by hydrophobic guiding elements.

Figure 6 is a sectional view of another embodiment of a detection area of the sample distribution system using hydrophilic pathways.

Figure 7 shows the schematic principle of an agglutination reaction: A) introduction of the fluid sample medium, B) dissolution of recognition and calibration formulation, C) finally formed agglutinate between analyte, recognition element, and calibration compound.

Figure 8 shows the schematic principle of agglutinating microparticles: A) introduction of the fluid sample medium, B) dissolution of recognition and calibration formulation, C) finally formed agglutinate between analyte, recognition element immobilised on microparticles, and calibration compound.

Figure 9 shows different embodiments of the sample distribution system with different patterns of pathways and detection areas suitable for different calibration methods.

Figure 10 shows a sample detection area of the sample distribution system of Fig. 6 in conjunction with a light emitter and detector means in a sectional view configured for absorbance measurements.

Figure 11 shows the detector arrangements for the analyte test element configured for the evaluation of light scattering or fluorescence.

Figure 12 shows schematically the results and evaluation of a qualitative analysis of non competitive A) and competitive assays B).

Figure 13 exemplifies the determination of the analyte concentration using the linear standard addition method.

Figure 14 provides a graph showing the validation method for the calculated result and calibration data.

Figure 15 shows examples of fluorescent dyes with unspecific protein affinity.

Figure 16 shows the schematic principle of an agglutination reaction enhanced by a fluorescent molecular rotor: A) introduction of the fluid sample medium, B) dissolution of recognition and calibration formulation, C) finally formed agglutinate between analyte, recognition element, calibration compound, and unspecific fluorescent molecular rotor as reporter element.

Figure 17 shows examples of unspecific fluorescent molecular rotors.

Figure 18 shows the schematic principle of an affinity reaction employing specific fluorescent dye as reporter element: A) introduction of the fluid sample medium, B) dissolution of recognition and calibration formulation, C) finally formed affinity complex between analyte, recognition element, calibration compound, and the specific reporter element.

Figure 19 shows the reaction principle of an apo-enzyme assisted recognition assay (AARA).

Figure 20 shows the influence of registration failures during the lamination process on the sample volume of the analyte test element as well as the top respectively the sectional view of an alternative embodiment c), which allows higher tolerances for the registration of base and cover layer without compromising on the test strip quality.

Figure 21 shows a preparation method of an analyte test element useful for affinity and immunoassays.

## Detailed Description of the Invention

As shown in Figure 1 and Figure 2, the analyte test element 1 of the present invention is a multiple layer arrangement comprising a base layer 2, a centre layer 3 overlaying the base layer 2, and a cover layer 4 overlaying the centre layer 3. The centre layer 3 presents a discontinuity 5, which creates a hollow cavity in conjunction with the base layer 2 and the cover layer 4. Within said cavity there is located a sample distribution system 6 which is connected to a sample application area 9 located on one side of the analyte test strip. The sample application area 9 as interface to the user is preferably formed by a convex curve 10 extending from one major side of the analyte test strip for easier application of the sample. Opposite to the sample application area 9, 10 on the second major side of the analyte test strip is the location of an air vent 11 allowing the displacement of air while the physiological or aqueous fluid is distributed to the predetermined detection areas 6a, 6'a (see Figure 3). It might be noted that the construction requires only one air vent independent of the amount of predetermined detection areas used within the analyte test element. The described elements of the sample distribution system with areas of high surface energy, sample application area, air vent, centre layer and discontinuity in the centre layer form the totality of the analyte test element, which creates the intrinsic capillary action to exert the distribution of the applied physiological or aqueous fluid to the predetermined detection areas.

In addition, the analyte test strip 1 possesses registration features 7, 8 useful to differentiate between several kinds of analyte test elements for the determination of different analytes. By this means, a multi-analyte meter could be instructed to run a special program or procedures with selectable parameters upon strip insertion required for the determination of a particular analyte.

As illustrated in Figure 3, which represents the multi-layer arrangement of Figure 1 and 2 in an exploded view, the base layer 2 provides a first surface 2a, and the cover layer 4 provides a second surface 4a. The first surface 2a

and the second surface 4a are patterned with areas which will create the sample distribution system 6. The pattern of the sample distribution system 6 comprises a predetermined number of analyte detection areas 6a and sample pathways 6b, which are aligned and registered mostly congruent upon assembly of the multi-layer arrangement. The centre layer 3 defines the distance between the first surface 2a of the base layer 2 and the second surface 4a of the cover layer 4 and has a discontinuity 5 to form a hollow cavity together with the first surface 2a of the base layer 2 and the second surface 4a of the cover layer 4. The sample distribution system 6 which will be formed between the first surface 2a and second surface 4a is located within the cavity created by the discontinuity 5 of the centre layer 3 and the first surface 2a of the base layer 2 and the second surface 4a of the cover layer 4. Preferably, the hollow cavity is substantially larger by design than the sample distribution system.

Since the purpose of the discontinuity 5 of the centre layer is only to create a cavity for the sample distribution system 6, the discontinuity 5 of the centre layer 3 can have different forms; examples thereof are shown in Figure 4. Figure 4a shows an umbrella shaped analyte test element cavity 12, Figure 4b shows a rectangular analyte test element cavity 13, and in Figure 4c the sample cavity 14 has a circular shape. The discontinuity 5 of the centre layer 3 does not influence the size of the predetermined detection areas 6a and the size of the pathways 6b of the sample distribution system 6 and therefore does not influence or change the required sample volume. Compared to the sample distribution system 6, the cavity shapes shown in Figure 4 are rather simple, thus allowing the application of simple punch tools and fast processing with less demand on the registration accuracy.

The sample distribution system 6 located in the cavity formed by the discontinuity 5 of the centre layer 3 and the first surface 2a of the base layer 2 and the second surface 4a of the cover layer 4 is formed by creating areas of high and low surface energy on said surfaces 2a and 4a. The areas of high and low surface energy on the first surface 2a of the base layer 2 and the second surface 4a of the cover layer 4 are aligned and registered mostly congruent to each other. Since the applied physiological fluid or any other aqueous sample is wetting only the areas with high surface energy, it is thus constrained within the predetermined flow paths 6b and detection areas 6a of the sample distribution system 6 and between the first surface 2a of the base layer 2 and the second surface 4a of the cover layer 4.

Figure 5 shows a construction of the sample distribution system using hydrophobic "guiding elements". In this embodiment of the analyte test element of the present invention the base layer 2 and the cover layer 4 are coated with a hydrophobic layer 16, except the areas, which will form the sample pathways and detection areas. The hydrophobic layer 16 creates an area with low surface energy, which will exert a repellent force onto an applied sample fluid 15 and constrain the sample fluid 15 therefore to the areas of high surface energy which will form the sample distribution system.

Preferably, the hydrophobic layer 16 is applied on a hydrophilic surface 2a, 4a, which is wettable by the physiological or aqueous sample fluid 15. The procedure described above requires a hydrophilic surface, which can be produced from a natural hydrophilic polymer such as cellophane or glass as well as from a hydrophobic surfaces of common polymers (examples are given below) by rendering the hydrophobic surface hydrophilic using a coating process or physical or chemical plasma deposition of hydrophilic monomers that can be vaporised in vacuum, e. g. silicon dioxide, ethylene oxide, ethylene glycol, pyrrole or acrylic acid. Subsequently, the pattern of "guiding elements" can be realized by printing hydrophobic ink on the hydrophilic surfaces of the base and cover layers.

A suitable hydrophobic ink will have contact angles with water of typically more than 100° and a surface energy of typically less than 25 mN/m and contain typically monomers, oligomers, and polymers with hydrophobic functions, hydrophobing additives, or hydrophobic pigments and fillers.

Figure 6 shows another construction of the sample distribution system using hydrophilic pathways. In this embodiment of the analyte test element the naturally hydrophobic base layer 2 and cover layer 4 are coated with a hydrophilic compounds or varnish 17 thereby creating areas of high surface energy.

The hydrophilic agent 17 printed on the hydrophobic surface 2a, 4a is highly wettable by a physiological or aqueous sample fluid 15; thus, the areas of high surface energy creating the hydrophilic pathways of the sample distribution system will exert a positive capillary force onto the applied physiological or aqueous sample fluid 15 to transport the sample fluid to the separate detection areas.

The hydrophilic pattern can be realized by printing hydrophilic or amphiphilic agents on a hydrophobic surface. Inks with hydrophilic functions can be realised from a wide selection of high molecular weight polymers and mixtures thereof, soluble in polar solvents e.g. water or alcohols. Particularly useful are derivatives prepared from alginate, cellulose and cellulose ester, hydroxyethyl cellulose, gum, acrylic acid, polyvinyl-alcohol, polyethylene-glycol, polyethylene-oxide, polyvinylpyrolidone, polystyrene sulfonate, poly(methyl vinyl ether/maleic acid), vinylpyrolidone/trimethylammonium copolymers, and alkyl-phosphocholine derivates. Further optimisation can be achieved with organo-modified silicone acrylates additives, which are a cross-linkable species of organo-modified polysiloxanes, and fluorinated surfactants. Suitable coatings provide a contact angle with water of typically less than 35° and a surface energy of typically more than 50 mN/m.

The base layer 2 and cover layer 4 suitable as substrate for the printing process may be formed of a material like glass, polyvinyl acetate, polymethyl-methacrylate, poly-dimethyl-siloxane, polyesters and polyester resins containing fluorene tings, polystyrenes, polycarbonates and polycarbonate-polystyrene graft copolymers, terminal modified polycarbonates, polyolefins, cycloolefins and cycloolefin copolymers, and/or olefin-maleimide copolymers.

In case the substrate has an intermediate hydrophobic character, the printing of hydrophilic pathways with a surrounding hydrophobic pattern, i.e., a combination of the constructions of Figure 5 and Figure 6 is possible as well.

In an alternative embodiment, either the first or second surface is provided with the hydrophilic/hydrophobic pattern (6, 16) whereas the corresponding surface provides a homogeneous pattern of hydrophilic pixels surrounded by a hydrophobic area thereby creating a surface with semi hydrophilic and semi hydrophobic character (amphiphilic character), which eliminating the necessity to align the hydrophilic and hydrophobic patterns (6, 16) of the first surface with an equivalent hydrophilic and hydrophobic pattern (6', 16') of the second surface. The properties of such an amphiphilic surface can be easily designed by the geometric pattern of the hydrophilic pixels and the overall ratio between the hydrophilic and the hydrophobic area. In the disclosed invention the amphiphilic character, respectively the ratio between hydrophilic pixels and hydrophobic areas, is designed that the sample fluid progresses from hydrophilic pixel to hydrophilic pixel only if the opposite surface provides hydrophilic character. If the opposite surface provides hydrophobic character the movement of the fluid within the capillary gap of the analyte test element will stop. This mechanism allows the above-described method to form a functional analyte test element without the stringent requirement of precise registration of the corresponding pattern of the sample distribution system provided on the first and second surface.

However, preferably both the first and the second surface are provided with equivalent patterns of high and low surface energy to ensure a quick distribution of the sample fluid within the hydrophilic pathways of the sample distribution system.

Moreover, it is possible to physically elevate the areas of high surface energy of first and second surfaces from the areas of low surface energy by etching, embossing, or simply by printing the hydrophilic layer 17 with about three to five times increased thickness on the first and the second surface. Due to this elevation the capillary gap of the hydrophilic pathways gets smaller in relation to the surrounding area and exerts a higher capillary forth on the sample liquid.

The volume requirement for the sample distribution system contained in the analyte test element of the preferred embodiment is with about 0.5μL-1.0μL very low and requires only about 100nL - 150nL per detection area, whether the areas of high and low surface energy are created by hydrophobic guiding elements or hydrophilic pathways or by a combination of both. However, it will be obvious for the one skilled in the art that the volume of the sample distribution system will vary with various designs and with the number of employed predetermined detection areas.

As shown in Figures 5 and 6, the areas of high surface energy of the first and second surfaces 2a, 4a forming the detection areas are coated with formulations 18, 19 promoting the detection and allowing the determination of an analyte in the sample fluid 15. In the embodiment shown in Figures 5 and 6 the first surface 2a of the detection area is coated with a calibration formulation 18. The calibration formulation contains a calibration compound which can be the analyte or an analyte equivalent molecule which has an affinity to the recognition element The second surface 4a of the detection area is coated with a recognition formulation 19 containing a recognition element, e. g. a receptor, an antibody or antibody fragment such as a Fab fragment, which has an affinity to the analyte and which is able to promote the detection and determination of the analyte in the sample fluid. Specific examples of the calibration compound as well as of the recognition elements are given below.

As mentioned previously, depending on the need of the physician and the test to be performed, different diseases prevalent in a body require different sensitivities and dynamic ranges, i.e., an analyte test system for HIV will require a very sensitive approach for threshold detection but no wide dynamic range, whereas the detection of HbA1c, a 3 month marker for the development of diabetes and the compliance of a patient, requires a wide dynamic range but no extreme sensitive threshold.

Further, the onset of diabetes complications such as diabetes retinopathy requires a very sensitive approach when using a protein based detection means. For instance the detection of such a complication can at least be a two-fold exercise i.e. traditionally, the onset of retinopathy is monitored by ophthalmologists and the diagnosis and condition of diabetic retinopathy is made following a detailed examination of the retina with an ophthalmoscope. In a second detection technique of diabetes retinopathy, the protein concentration of Stromal cell-Derived Factor-1 (SDF-1) is known to increase in relation to the progress of diabetic retinopathy correlating with disease severity. Such a protein can therefore be used as a basis for an assay for the detection and monitoring of a disease complication such as retinopathy.

Thus, the detection methods and regimes have to be adapted to the requirements of diagnostic needs respectively the therapy requirements of the patient. The following disclosed and discussed examples are preferred but not the only implementations of the analyte test element to fulfil the required diagnostic task. They contain in most cases chemical and/or biochemical compounds such as fluorescent dyes, microparticles, coenzymes, and apo-enzymes allowing or enhancing the detection of a desired affinity reaction (recognition reaction) between the analyte and a suitable analyte specific recognition element, thus one can refer to these compounds generally as reporter elements or in its plurality as reporter system.

## Analyte test systems utilising reporter elements for the analysis of affinity assays

### Agglutination methods

Figure 7 shows a schematic view and principle of a first embodiment of the present invention applicable for affinity and immunoassays or generally stated for recognition assays. In the current embodiment an agglutination reaction is described which indicates the qualitative respectively quantitative reaction between an analyte of interest 31 and a specific recognition element 32 thereof. In this particular case the analyte 31 could be a protein such as a virus protein or hCG, and the recognition element 32 could be an antibody against this protein.

Figure 7 shows schematically the reaction if a liquid sample 15 containing the analyte of interest 31 entering the sample detection area containing the recognition element 32 and calibration compound 33 attached to the first and second surfaces 2a and 4a. The recognition element 32 as well as the calibration compound 33 are solved by the liquid sample fluid 15 and the recognition element 32 reacting with the calibration compound 33 as well as with the analyte 31 contained in the sample fluid. For the reaction to progress the recognition element 32 is able and needs to bind to more than one analyte molecule, thus the recognition elements 32 will subsequently cross-link or agglutinate all analyte molecules to large aggregates 34. The size of these aggregates or particles will cause LASER light to scatter dependent on their physical size. If the aggregate is smaller than the wave length of light the aggregate will produce a particular type of scattering known by one skilled in the art as Rayleigh respectively as Rayleigh-Gans-Debye scattering, whereas aggregates larger than the wave length of light will induce scattering known as Mie scattering. The different types of scattering, the pattern of the scattering in relation to the LASER light wave length can be used to determine the existence respectively the quantity of the analyte.

In case the recognition element or elements 32 is/are immobilised on microparticles 35 made of e.g. gold, latex, or polystyrene, as shown in Figure 8, the agglutination reaction between a plurality of such sensitised microparticles and the analyte can be followed by a simple photometer or even by eye. During the progress of the agglutination reaction the amount of single microparticles is reduced by agglutination, thus the number of scatter centres in the sample is reduced and the light is scattered less after the discussed reaction, allowing a higher transmission of light through the sample detection area.

Likewise, the absence of an affinity reaction between the analyte and the recognition element in a liquid sample medium is distinguished by the absence of an agglutination reaction and therefore characterised by a low transmission of light through the sample detection area.

The analyte test element is constructed by coating the detection areas 6'a of the first surface 2a of base layer 2 with a suitable recognition element 32 respectively sensitised microparticles 35. Preferably, the microparticles 35 can be latex, gold or gelatine, but more preferably gold. Other known particles such as carbon, polystyrene, or the like can be used which would be obvious to persons skilled in the art. Alternatively, the microparticles 35 can be coated on the detection areas 6a of the second surface 4a of a cover layer 4. Furthermore, the microparticles can be coated both on the detection areas of said first surface 2a and on the detection areas of the second surface 4a as long as no calibration formulation 18 respectively calibration compound 33 is required for the analysis of the analyte.

In another and more preferred embodiment the detection areas 6a of said first surface 2a are coated with calibration formulations 18 allowing a positive and a negative control analysis to be performed in parallel to the determination of the analyte thus providing the possibility to give a user assurance that the measurement is performed correctly and that the analyte test system as well as the applied analyte test element was fully functional. Such manifold provides a high concentration of calibration compound (analyte or an analyte equivalent molecule which has an affinity to the recognition element) in one of the detection area e.g. $6'a_3$ which allows full completion of the agglutination reaction. Thus, the reading of detection area $6'a_3$ would relate to approximately 100% or full scale reading of a meter device. A second detection area e.g. $6'a_2$ holds an inhibitor compound prohibiting the affinity reaction of the analyte 31 with the recognition element or a non reactive recognition element such as non sensitised microparticles thus relating the reading of detection area $6'a_2$ to approximately 0% of the agglutination reaction. In this embodiment the concentration of the recognition element respectively sensitised microparticles in all predetermined detection areas 6a will be equal representing an optimized concentration for the particular assay as optimised in the laboratory.

In a further and most preferred embodiment two of the detection areas of the first surface 2a of the base layer 2 are coated with calibration formulations 18 containing different and predetermined concentrations of the analyte or compounds inducing the same or an equivalent non-catalytic affinity reaction with the recognition element.

In the most simplistic calibration model the optical response caused by an affinity reaction between the analyte and the recognition element is linear or can be easily linearised by a suitable mathematical function such as f(log(x)) or f(1/x). After introduction of the liquid sample using the sample application area 9 the sample is distributed to the sample detection areas. Here the sample solubilises the calibration formulation 18 coated on the first surface 2a and the recognition formulation 19 coated on the second surface 4a initiating the affinity reaction between the analyte 31, the calibration compound 33, and the recognition element 32. Due to the different concentration of calibration compound in

the detection areas 6'$a_2$ and 6'$a_3$ which are added to the analyte contained in the sample the meter respectively the detection device can relate the optical reading of all detection areas 6a to the concentration of the analyte in the sample fluid. Optionally, sample detection area 6c will contain no recognition element or non-specific binding partner such as non sensitised microparticles, thus the application of sample fluid in detection area 6c will trigger no agglutination and is useful to evaluate background signals of the sample fluid 15 in the analyte test element. The discussed method of calibration is known to one skilled in the art as "standard addition method" and is used in laboratories for samples with "difficult" sample matrices.

Figure 9 shows different patterns of the sample distribution system, which can be realized by hydrophilic pathways as illustrated in Figure 6, or by the hydrophobic "guiding elements" as illustrated in Figure 5, or by a combination of hydrophilic pathways and hydrophobic guiding elements. Cell AI in Figure 9 illustrates all cases for the simplest sample distribution system. Column A of Figure 9 shows the formal design of sample distribution systems with no background correction, whereas column B provides designs for sample distribution systems with background corrections, column C indicates the highest order of the polynomial calibration equation achievable with the adjacent designs, and column *n* indicates the required number of predetermined detection areas of each surface, respectively the number of required measurements. The literals in each design indicate the position of the background correction (c), sample (1), and all associated calibration areas (2, 3, 4, 5, 6) with increasing amount of calibration compound. The simplest calibration is represented by a linear equation where the relationship between measurement and the analyte concentration is strictly proportional. The calibration of the analyte test element is generally performed using the standard addition method by adding the calibration compound of the different calibration areas to the sample and subsequent calculation of a linear or monotone non-linear calibration equation. Figure 13 gives a more detailed explanation about case I. The calibration model or order (column C) needs to be appropriate for the selected analyte and employed detection chemistry, consequently it is not possible to apply a linear calibration model to a chemical reaction which obeys a fourth order model and vice versa. However, it is still possible to use the analyte test element designed for five standard additions for a linear calibration, the higher amount of standards will allow an even more precise measurement and a statistical validation with higher significance in terms of correlation coefficient, standard deviation and standard error of the test compared to a linear calibration based on two standards.

Moreover, the repetition of sample and standard measurements is possible as well, thus it is possible to perform two independent linear calibrations for one particular sample of physiological or aqueous sample fluid with the embodiments shown in row IV. Likewise, it is possible to use the same analyte test element for the determination of two analytes. Additionally, a multi analyte system can be realised within the same set of predetermined detection areas if the selected detection chemistries generates no interference problems, thus the reaction educts and products of one reaction will not take part in the other reaction and the produced indicator dye absorbs the light in a substantially different wave length range.

In a further and alternatively embodiment of the analyte test element, the calibration is more of a qualitative nature. Here, the result of the detection area 6$a_1$ is compared with the detected limits of a positive standard 25c and negative standard 26c provided in additional detection areas e.g. 6$a_2$ and 6$a_3$ (see Figure 12 for details). Such method is useful for analytes requiring only a qualitative indication generally known from pregnancy test systems.

In case the optical response of the reaction between the analyte and the recognition element is not linear, other calibration models can be employed if the sample distribution system is adapted according Figure 9 to a non linear behaviour by introducing more predetermined sample detection areas in the sample distribution system of the analyte test element. One skilled in the art will note that Figure 9 gives an exemplary collection of applicable sample distribution systems for the purpose of illustration. Other geometries might be also possible and useful to fulfil equivalent or related tasks.

The optical response of the reaction between the analyte and the recognition element 32 contained in the recognition formulation 19 is measured by a meter or detection device. The meter or detection device provides a strip holder with at least one but preferably a plurality of arrangements containing a light source 20 to emit a light wave 24 through a predetermined sample detection area 6 as shown in Figure 10. The wavelength of light wave 24 can be in the range of 300 to 800nm, but is preferably between 400 and 650nm and most preferably 635 nm. The light emanating from said source passes through an optical arrangement 21, 22 such as a lens and an aperture, the base layer 2, liquid medium 15, the cover layer 4 of the detection area and is detected on the opposite side of test element by a detection means 23. Preferably, the light detection means includes an optical arrangement 21a and 22a such as an aperture, lens and/or an optical filter and at least one of a photodiode, phototransistor, photocell, photomultiplier or an array of charge-coupled devices. More preferably the light detection means comprises a photodiode. The light source could be a light emitting diode or a LASER diode with a suitable wavelength.

In case the agglutination reaction is monitored directly by light scattering and without the aid of microparticles it is most useful to arrange the light source and the detection mean not opposite each other but rather in an angle of approximately 90 degrees to achieve maximum sensitivity. The preferred angle between the light source and the detection means is between 80 and 120 degrees but most preferably it is approximately 109 degrees and the analyte test element

is placed in the optical detection arrangement in a way that the angle between the base layer and the light source and the angle between the cover layer and detection means is approximately 54 degrees to reduce background noise due to internal reflections on the different surfaces of the detection area (or more generally of the analyte test element) as shown in Figure 11. Thus, the detection means will only see the light wave 24 diverted by the scattering on the agglutinated particles and not the light wave 24a originated by the light source. Albeit, one skilled in the art will notice that the actual angle has to be optimized for a specific application, the required sensitivity, and the requirements of the meter respectively the detection device. Further more, the arrangement shown in Figure 11 can be employed for fluorescence detection if the light source 20 and the photo detector 23 are configured with additional filters discriminating between the excitation and the emission wave length.

Again, the application of light waves into each sample detection area 6 during the occurrence of an affinity reaction prompts the scattering of the light waves. Since the concentration of analyte on each detection area is dissimilar following the addition of analyte or the addition of an analyte equivalent compound as calibration compound 33, the optical density of each resulting light beam is also dissimilar. In case there are at least three detection areas arranged within the sample distribution system, whereby at least two of the detection areas contain different levels of calibration compound, it is possible to calculate the unknown concentration of an analyte 31 in a liquid sample medium 15 in a predetermined detection area without calibration compound ($6'a_1$) as long the relationship between agglutination and the concentration of the analyte follows a linear or a monotone increasing respectively decreasing model. Potential sample distribution systems corresponding calibration models based on polynomial relationship are shown in Figure 9 as described above.

In the case of no occurrence of an agglutination reaction i.e. no reaction between the analyte 31 and the recognition element 32 in a sample detection area, the light will be scattered strongly leading to a pseudo high optical density (absorbance). Conversely, in the case of ~100% agglutination according to a high reaction between the analyte respectively the calibration compound and the recognition element on the first surface of a base layer and the second surface of a cover layer the light will be scattered minimally leading to a low optical density.

Figure 12 shows exemplary graphs of absorbance versus concentration with a qualitative detection regime. Here, the predetermined detection areas $6a_2$ and $6a_3$ are configured to provide information of the dynamic range of the agglutination reaction, said $6a_2$ provides a comparison to ~0% agglutination representing a negative standard 26c, respectively $6a_3$ provides a comparison to ~100% agglutination representing a positive standard 25c, thus the readings of these detection areas can be used to validate the measurement performed in detection area $6a_1$.

Figure 13 shows exemplary graphs and evaluations of absorbance versus concentration of a agglutination reaction between the analyte and the recognition element in a liquid medium suitable for quantitative analysis. The concentration of the recognition element, which might be for example a predetermined amount of antibodies or antibodies immobilised on microparticles, on the detection areas $6a_1$ to $6a_3$ is optimised to give an appropriate sensitivity and dynamic range for the analyte of interest. Furthermore, in a fourth and optional detection area, the light scattering or optical absorbance of the sample located in detection area 6c is measured. The readout of this detection area represents a quantification for the sample background providing an integral information of the unspecific portion of the reaction generated by the materials of the analyte test element such as firms, non active coating compounds, unspecific proteins, or non-sensitised microparticles, or the sample matrix itself, which is especially required for a quantitative sample evaluations.

As previously mentioned, the coating of the calibration formulation 18 containing the calibration compound 33 and of the recognition formulation 19 containing the recognition element 32 respectively the recognition element immobilised on suitable microparticles 35 can be performed by several known techniques such as micro-contact printing, drop on demand printing or deposition methods, or other capillary contact deposition methods. The concentration of the active components in the coatings or inks such as the calibration compound and the recognition element within the analyte test element can be optimised by laboratory methods to define an appropriated dynamic range and sensitivity of the analyte test system for a specific analyte of interest.

If the dynamic range of a specific test is evaluated in the laboratory the determined characteristics can be programmed in a meter or detection device as a validation window 29, as shown in Figure 14 to perform a validation of the results subsequent to the measurements and in addition to the evaluation of the statistical parameters available from the calibration calculation 30 such as standard deviation, standard error, and regression coefficient

**Fluorescence assisted detection methods**

The performance of the above described approaches for agglutination assays is dependent on the assay speed and the economy of the measurement device, both of these parameters being interrelated. On a molecular scale microparticles are huge and approximately 1000 to 10,000 times larger than an IgG antibody, which is a very common example for the recognition element. In case of the recognition element(s) 32 being immobilized on microparticles 35, the diffusion of the sensitised microparticles will be much slower than the diffusion of the recognition element alone effecting the

assay time and therefore the performance of a potential product, but the agglutination reaction itself could be monitored with very simple and cost efficient detection means or even by eye, if the appropriated microparticles are chosen, due to the fact that the scattering intensity will increase about $10^{20}$ if the particle size is increased just by the factor of 5.

On the other hand, the agglutination reaction between the analyte and a non-immobilized recognition element 32 can be successfully monitored on a molecular level with the right LASER assisted monitoring devices as described above. It is even possible to quantify the molecular weight of the recognition element and the resulting agglutinate after the reaction with the analyte. However, the cost for the necessary equipment, mainly due to the employed LASER technology, prohibits the use in point of care or home-monitoring applications.

A further aspect of the disclosed invention provides a solution which will take the middle ground between the two described agglutination approaches. On a molecular level agglutination of the recognition element and the analyte produces a mesh of small protein particles changing the partial molarity between water and proteins, even though the total mass balance between proteins and water will not be affected. After the agglutination reaction certain domains in the sample fluid will have higher concentrations and certain domains will have lower concentration of proteins compared to the point in time prior to the agglutination reaction. This change in physical properties can be detected and reported either by fluorescent quenching of dye molecules which have a high affinity to proteins or by fluorescent molecular rotors.

**Fluorescent dyes with unspecific protein affinity as reporter element**

The underlying principle of this detection method utilizes the effect of concentration dependent fluorescence quenching. Above a certain concentration fluorescent dye molecules become scavengers for the fluorescence emitted by other fluorescent dye molecules, thus the total detectable emission of the sample will decrease with concentration. This decrease depends on the nature of the fluorescent dye and the direct distance between two fluorescent dye molecules, in other words, the closer the dye molecules and the shorter the distance becomes the stronger will be the fluorescence quenching. In a homogenous solution the described and well known fluorescence quenching effect will be simply driven by the concentration of the fluorescent dye, whereas the quenching threshold will be dependent on the provided fluorescent dye or mixtures of fluorescent dyes. The quenching effect becomes only interesting for recognition assays if the dyes are not homogenously distributed in the sample fluid and somehow reflect the agglutination of the analyte and the recognition element resulting in a non homogenous distribution of analyte and recognition elements in the sample fluid. Additionally, it is beneficial to select fluorescent dyes which will show an increased fluorescent quantum yield only after the dyes become adsorbed onto the protein surface to minimise the background signal. Suitable candidates for this assay principle can be found in the class of amphiphilic zwitterionic aniline-pyridinium dyes with two homologous series derived from dibutylaninlino-pyridinium-butylsulfonate, and stryryl dyes (also known as Hemicyanine dyes) such as RH364, RH160, RH237, RH421, di-4-ANEPBS, BNBIQ, ANNINE-5, and ANNINE-6. Structure formulas of these exemplary dyes are provided in Figure 15. However, more commonly known dyes such as Nile Blue A, Phloxine B, 1-anilino-8-naphthalene sulfonate (ANS), or their derivates could be used for the described method as well.

The key reaction of this approach is the in-situ coating of the recognition element and analyte inside the liquid sample medium with a selected dye, thus the reaction is limited to recognition elements and analytes which basically belong to the family of proteins. However, the sequence of events, agglutination reaction and dye adsorption, is not of importance as long as the dye adsorption is not interfering with the agglutination progress. During the agglutination progress the partial molarity of the dye increases within the agglutinate and induces the quenching effect. The concentration of the dye provided in the analyte test element has to be evaluated for every dye, recognition element, and analyte combination as well as for other experimental conditions but is generally depending on the dye protein coverage, dye protein binding constant, compactness of the agglutinate, and the overlapping of excitation and emission integral of the dye, which is a function of the stokes shift and half bandwidth of the fluorescence spectra of the dye.

After the application of the liquid sample medium onto the sample application area 9 the sample is distributed by the sample distribution system 6 to the sample detection areas 6a. Here the sample dissolves the calibration formulation 19 containing the fluorescent dye and the calibration compound 32 on surface 2a of the first layer 2 and the recognition formulation 18 containing the recognition element 32 on surface 4a of the second layer 4. After the mixing of the provided compounds of the first and the second surfaces the reaction begins whereby the recognition element and the analyte, provided the analyte is a protein, becomes covered with the dye and the population of recognition element molecules respectively particles undergoes cross linking with the analyte. Thus, the amount of fluorescence quenching can be measured by the detection means of the analyte test element. For qualitative detection regimes two of the sample detection areas are configured as negative control $6'a_2$ and positive control $6'a_3$. The measurement of the negative control will relate to the fluorescence of the entire protein content of the sample plus added ingredients provided on the involved sample detection areas, without agglutination. To avoid the agglutination reaction, detection area $6'a_2$ is coated with an unspecific recognition element without affinity to the analyte. Consequently, the fluorescence reading of this detection area will result in the 100 % signal characterising the upper limit of the dynamic range.

Conversely, the measurement of the positive control arranged in sample detection area $6'a_3$ will relate to the

fluorescence of a fully formed and agglutinated dye aggregate providing the maximum of fluorescence quenching possible. Such positive control can be arranged by an excess of calibration compound respectively analyte or analyte equivalent with in the calibration formulation coated on sample detection area 6'$a_3$. This configuration will not only allow to report the presence or the absence of an analyte but also give the opportunity to report semi-quantitative information about the analyte concentration in relative terms compared to the negative and positive control measurements such as low, medium, and high.

If the relationship between analyte concentration and fluorescence quenching is linear in the analyte concentration range of interest or could be linearised with a suitable mathematical formula, quantitative results for an analyte could be achieved if a known amount of calibration compound is deposited into the detection areas 6'$a_2$ and 6'$a_3$, whereby the concentration of calibration compound in detection area 6'$a_3$ is larger as the concentration of calibration compound in 6'$a_2$. In case this relationship is not linear, quantitative information of the analyte can still be gained with analyte test elements providing a higher number of sample detection areas and more than two different concentration levels of calibration compound (Figure 9). The method used by the processing means of the device to calculate the quantity of the analyte concentration by the linear standard addition method is identical for both examples apart from the signal pre-conditioning and scaling as given in Figure 13A and B. Thus, a more detailed elaboration of the standard addition methods is omitted in this example.

**Unspecific fluorescent molecular rotors as reporter elements for agglutination assays**

The progress on an agglutination reaction resulting in an agglutinate 34 with constrained fluorescent molecular rotors is given in Figure 16A to C. In this case the analyte test system exploits the effect that fluorescent molecular rotors 36 will increase their fluorescence if the rotation of special groups in the molecular structure is hindered either by the increase of viscosity or by steric hindrance. Accordingly, the fluorescence quantum yield of the used dye will increase with the size of the agglutinate and the cross-linking level, whereas both parameters are functions of the analyte concentration.

Typical fluorescent molecular rotors are based on the main structure of xanthene dyes. Xanthene dyes are derived by condensation of phthalic anhydride with resorcinol (and derivatives) or m-aminophenol (and derivatives), of which fluorescein is the prototype (all such dyes have the xanthene nucleus). However, not all xanthene dyes are fluorescent molecular rotors. Although fluorescein provides the basic structure for a molecular rotor it does not provide the desired dependency and relationship between fluorescence and intermolecular rotation of side groups. This effect is not prevalent in fluorescein due to the hydroxyl side groups in 3' and 6' position of the molecule, which will be only effected by the pH of the sample medium and not by the micro viscosity of the sample fluid.

Effective fluorescent molecular rotors are based on the underlying xanthene structure prevalent in rhodamine dyes. These dyes are derived from condensation of phthalic anhydride with m-dialkylaminophenols. The hydroxyl groups (HO-) of the fluorescein are replaced in the rhodamine dyes with secondary amine groups ($R_2N$-) as effective rotors in the molecular structure. Rhodamine B, sometimes called Acid Red 52, has two ethyl groups as residues R in the molecular structure of the rotors and provides the required characteristics between fluorescence and molecular mobility to be employed in the analyte test system.

Rhodamine B, or Sulforhodamine B with increased water solubility, is only an exemplary fluorescent molecular rotor for the discussed application. Auramine O, Crystal violet, p-N,N-dimethylaminobenzonitrile, p-N,N-dimethylaminobenzylidenemalononitrile, Julolidinebenzylidenemalononitrile, Rhodamine 19, Rhodamine 6G, Oxazine 1, Oxazine 4, Oxazine 170, Rosamine dyes, Carbopyronine dyes, Pyronine dyes, and Thiazine dyes are further examples of effective fluorescent molecular rotors. Typical molecular structures are shown in Figure 17.

To achieve the desired dynamic range and sensitivity with the analyte test system respectively to optimally trace the reaction between the analyte of interest and the corresponding recognition element a most suitable fluorescent molecular rotor needs to be screened or even modified in respect to the size of the rotors. However, modifications of fluorescent molecular rotors will be within scope of the invention.

**Specific fluorescence reporter element using total molecular rotation for competitive and non competitive recognition assays**

In a further aspect of the invention the fluorescent dye takes part in an affinity reaction between the analyte and the recognition element, whereas a specific residue R of the dye has an affinity to the recognition element. In accordance to this approach all functional groups, some of the functional groups, or parts of the functional groups of the dye molecule are replaced by the specific residue R representing the structure of a reaction partner, e.g. an analyte, a peptide, a hormone, drug, or a nucleic acid such as an oligonucleotide or a short aptamer. The three examples below show possible modifications of the fluorescent dyes Fluorescein, Rhodamine 6G and Oxazin 4.

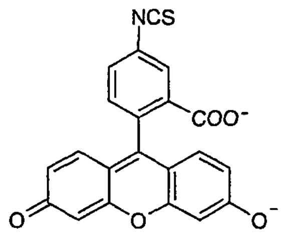

modified Fluorescein

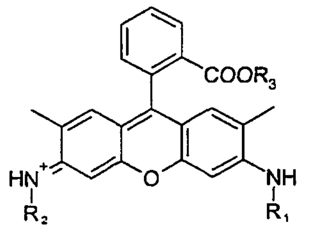

modified Rhodamine 6G

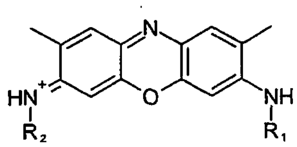

modified Oxazin 4

Contrary to the example of the fluorescent molecular rotor given above, where the change of the intra-molecular flexibility changes their fluorescence quantum yield, the now introduced detection scheme for the analyte test element focuses on the total molecular rotation of fluorescent dye molecules with specific affinity to the recognition element. It will be obvious to one skilled in the art, that the total molecular rotation in a given liquid sample medium is a function of the hydrodynamic molecular diameter, which is related to the molecular weight of the fluorescent dye. The total molecular rotation of fluorescent dye can be directly monitored by means of fluorescence polarisation, first discovered by Perrin in 1926.

The fluorescence polarisation is in general reverse proportional to the rotation speed of the molecule, thus the faster the dye rotates the lower will be the observed fluorescence polarisation signal, and therefore it is proportional to molecular weight respectively the apparent hydrodynamic molecular size. In other words, the larger the molecule the stronger will be the fluorescence polarisation signal.

To detect fluorescence polarisation, linear polarized light is used to excite the fluorescent dye of interest, and the resulting fluorescence emission is measured behind a second polarisation filter, whose polarisation plane is oriented either parallel ($I_{\parallel}$) or perpendicular ($I_{\perp}$) to that in the excitation device. When such linear polarized light is absorbed by the fluorophore, it can become depolarized, provided the molecule rotates in solution to a significant degree over the period of its fluorescence lifetime.

For example fluorescein, a fluorescent dye with a molecular weight of ~ 332 D, attached to ligands with a molecular weight below 5,000 D return only a weak fluorescence polarisation signal, due to a significant depolarisation attributed to a fast molecular rotation over the 4 ns of the fluorescence lifetime of the fluorophore. This means, during the second measurement with the filters oriented perpendicular to each other, there is considerable emission intensity passing through the detector, such that $I_{\perp}$ is significant and being a magnitude similar to $I_{\parallel}$. This results in a relative small polarisation signal.

In case the fluorescent dye binds to the large recognition element, the amount of molecular rotation over the fluorescence lifetime is strongly reduced and depolarisation of the signal is insignificant. Hence, a small emission intensity is measured with the polarisation filters oriented perpendicular to each other, making $I_{\perp}$ small, producing a relatively high polarisation signals result.

To observe the fluorescence polarisation the meter device needs to be configured with two polarisation filters one in the optical arrangement 21, 22 of the light source 20 and another one in the optical arrangement 21a, 22a of the optical detector 23. The meter device needs to measure fluorescence intensity twice. For one measurement, the polarisation filters are oriented parallel to each other ($I_{\parallel}$); the second measurement is acquired with the filters oriented perpendicular to each other ($I_{\perp}$). Prior art devices can select different polarisation planes with polarisation filters mounted in a wheel or other mechanical fixtures allowing the change over by rotation or physical movement. The mechanical selection of filters provides good flexibility and some advantage for laboratory equipment. However, it is not suitable for small and portable devices or even a hand held device.

A non-mechanical solution for this problem is the selection of the parallel or perpendicular polarisation plane by a liquid crystal filter or liquid crystal polarisation plane selector. The liquid crystal filter or liquid crystal polarisation plane selector can be understood as a sandwich construction of three carrier layers parallel to each other hosting in-between two layers of liquid crystals, which can be oriented into one polarisation plane by applying a potential to two adjacent carrier layers enclosing the liquid crystals. The potential to said two adjacent carrier layers can be controlled by a processing unit of a meter device. One skilled in the art will recognize the relation of the liquid crystal polarisation plane selector to a modified liquid crystal display unit.

The processing unit of the meter device can now calculate from the two measured fluorescence intensities from both polarisation planes to the fluorescence polarisation P by the following formula:

$$P = \frac{I_{\parallel} - I_{\perp}}{I_{\parallel} + I_{\perp}}$$

It becomes obvious from the formula that the polarisation unit P is dimensionless and therefore becomes independent from the concentration of the used fluorescent dye, the intensity of the light source, and most important from the attenuation of the fluorescence intensity due to the properties of the sample itself. This detection principle will be equally valid for competitive and non competitive recognition assays as discussed blow.

Competitive assays show a reverse proportional characteristic between the analyte amount and fluorescence polarisation, thus low concentrations of analyte give a strong fluorescence polarisation and high concentrations of analyte will give a minute fluorescence polarisation (as shown in Figure 12B). Additionally, the recognition element is considered as active binding element or host molecule such as an antibody and the analyte is considered as passive binding partner or guest molecule.

Conversely, a high concentration of analyte will always give a high fluorescence yield employing the above described agglutination approach with unspecific fluorescent molecular rotors, where the fluorescent molecular rotor is not directly involved in but constrained and hindered in rotation by the recognition reaction (as shown in Figure 12A).

The schematic progress of a reaction between the analyte 31 and the recognition element and a specific reporter element 37 is provided in Figure 18. After the sample introduction A) and dissolution of the recognition and calibration formulation containing the specific fluorescent dye as reporter element B) the recognition reaction C) takes place. Now the analyte 31 contained in the sample medium 15 competes with the specific residues of the fluorescent dye 37 for the available recognition elements provided on the second surface 4a of the sample detection area forming a complex mesh 38 between all reaction partners. If the concentration of analyte molecules is high almost all the binding sites of the provided recognition element 32 will be occupied, leading to none constrained freely rotating specific fluorescent dye 37 with a low fluorescence polarisation. Conversely, if the concentration of the analyte molecule in the sample medium is low the binding sites of the recognition element 32 will be mostly occupied by the specific residues of the fluorescent dye 37 thus leading to a constrained dye molecule and a high fluorescence polarisation. This general relationship of a competitive assay concept is illustrated in Figure 12B.

However, one skilled in the art might consider one further configuration where the analyte itself act as active binding element respectively as host molecule. This will be the case if the analyte of interest is an antibody i.e. if a patient is tested for his response to a vaccination. In this case the specific fluorescent dye reacts directly with the analyte without involvement of a further reaction partner, thus the reaction is non competitive and the fluorescence polarisation will be proportional to the concentration of the analyte over the dynamic range of the reaction. This situation is illustrated in Figure 12A.

The basic construction of the analyte test element follows the earlier given description. Again, the analyte test element can be configured to give qualitative results or quantitative results. Qualitative results will provide information of the presence or absence of an analyte, or information if the analyte is present in low or a high concentration, which can be achieved with a positive and a negative control measurement to determine the dynamic range of the analyte test system. Accordingly, detection area 6'$a_2$ is configured as negative control when the fluorescent dye is not constrained by the affinity reaction thus the detection means can evaluate the background fluorescence (in case of the competitive approach this will be the positive control). This state can be achieved by use of an unspecific recognition element in the detection area 6$a_2$.

Detection area 6$a_3$ is configured as positive control when the fluorescent dye becomes fully constrained either by the fully developed agglutinate or if the fluorescent dye is completely captured by the recognition element thus the detection mean of the analyte test system can register the maximum fluorescence (in case of the competitive approach this will be the negative control). Thus the calibration formulation contains enough analyte or calibration compound to trigger a fully completed affinity reaction.

Quantitative results are gained with the analyte test element if at least two sample detection areas are configured with at least two different concentrations of calibration compound with in the calibration formulation. The minimum amount of two different levels of calibration compounds would be sufficient to evaluate analytes with linear response characteristics. If such characteristic is more complex the numbers of sample detection areas respectively the number of different of calibration formulations has to be increased to fulfil the requirements of a suitable calibration model such as a higher order polynomial equation.

Fulfilling the requirements for a competitive linear model, the calibration formulation containing a specific fluorescent dye and a calibration compound, such as the analyte or an analyte equivalent molecule, is coated on the detection areas 6'$a_2$ and 6'$a_3$ of the surface 2a of the first layer 2. The calibration formulation coated on sample detection area 6'$a_1$ contains only a specific fluorescent dye as reporter element but no additional calibration compound. The

detection areas $6a_1$ to $6a_3$ on the surface 4a of the second layer 4 are coated with the recognition formulation containing the recognition element in a suitable concentration.

In case of non competitive approach the specific fluorescent dye becomes the recognition element and is coated on the detection area opposite to the calibration compound. For clarification the configuration for a linear competitive and linear non competitive analyte test system is given in the Table 1.

**Table 1** Compositions of the different coating formulations required for the different sample detection areas in competitive and non competitive assays

| Position in sample distribution system | Competitive Assay | Non Competitive Assay |
|---|---|---|
| **$6a_1$ (1st Surface)** | **Level 0 calibration formulation: only** specific fluorescent dye | **Level 0 calibration formulation** = **Blank formulation** |
| **$6'a_1$ (2nd Surface)** | **Recognition formulation:** actively binding component i.e. an antibody as recognition element | **Recognition formulation:** specific fluorescent dye |
| **$6a_2$ (1st Surface)** | **Level 1 calibration formulation:** analyte or equivalent with first concentration as calibration compound, specific fluorescent dye | **Level 1 calibration formulation:** analyte or equivalent with first concentration as calibration compound |
| **$6'a_2$ (2nd Surface)** | **Recognition formulation:** actively binding component i.e. an antibody as recognition element | **Recognition formulation:** specific fluorescent dye |
| **$6a_3$ (1st Surface)** | **Level 2 Calibration formulation:** analyte or equivalent with a second concentration as calibration compound, specific fluorescent dye | **Level 2 Calibration formulation:** analyte or equivalent with a second concentration as calibration compound |
| **$6'a_3$ (2nd Surface** ) | **Recognition formulation:** actively binding components i.e, an antibody as recogni tion element | **Recognition formulation:** specific fluo rescent dye |
| **6c** (**1st Surface)** | **Blank formulation** does not contain a fluorescent dye or recognition element against the analyte | **Blank formulation** does not contain a Blank formulation does not contain a fluorescent dye or calibration compound |
| **6'c (2nd Surface)** | **Blank formulation** does not contain a fluorescent dye or recognition element against the analyte | **Blank formulation** does not contain a fluorescent dye or calibration compound |

**Apo-enzyme Assisted Recognition Assay (AARA) as reporter element**

The Apo-enzyme Assisted Recognition Assay (AARA) technique can be utilised to detect the presence of an analyte in a liquid medium without a physical separation of free and bound species by washing, which is a common short-coming of immunoassays e.g. of Enzyme-Linked Immunosorbent Assays (ELISA) preventing the integration of this concept into a versatile test system. However, the AARA might be limited to small analytes such as hormones, steroids, peptides (e.g. B-type natriuretic peptide (BNP)), cardiac markers like troponin I (TnI), troponin T (TnT) and troponin C (TnC), haptens, organic molecules like pesticides, drugs (especially for drug of abuse applications: amphetamines, barbiturates, benzodiazepines, cocaine, methamphetamines, opiates, phencyclidine, THC), or explosives. Some of the known and utilised tumour markers in the diagnostic field e.g. Carcinoembryonic Antigen (CEA) with approximately 180,000 Daltons might be too large for this assay concept.

Figure 19 shows the principle mechanism of AARA employed in an analyte test system of the present invention having a recognition element 32 (e.g. an antibody), an analyte labelled co-enzyme 39 capable of reacting with an appropriate apo-enzyme 40 to form an enzymatic active holo-enzyme 41. Quantitative determination of an analyte by AARA exploits the fact that the concentration of the analyte 31 is proportional to the reconstituted concentration of the holo-enzyme respectively the modulated enzyme activity of holo-enzyme generated by the reconstitution of the analyte

labelled co-enzyme and the apo-enzyme.

In a specific example the co-enzyme could be the flavin adenine dinucleotide (FAD) coupled to a derivate of the analyte of interest, e.g. BNP, whereas the apo-enzyme of glucose oxidase is used as a part of the reporter element. In the absence of free analyte the labelled co-enzyme will react with the recognition element in this case a BNP specific antibody, thus prohibiting the reconstitution of the holo-enzyme. As more free analyte (e.g. BNP) is contained in the liquid sample medium a proportional ratio of the recognition element becomes occupied by the analyte and proportional more labelled co-enzyme becomes available for reconstitution of the holo-enzyme. For the described mechanism it is important that the labelled co-enzyme 39 becomes sterically shielded by the recognition element 32 preventing the reconstitution with the apo-enzyme 40. As acknowledged by someone skilled in the art, the activated GOD will begin to oxidise glucose to glucono-lactone and hydrogen peroxide, which can be used for a subsequent and suitable chromogenic reaction. The analyte test element is constructed with at least two but preferably three sample detection areas to implement the AARA. The construction follows the above description providing a base layer 2 and cover layer 4 in a predetermined distance opposing each other with a sample distribution system 6 on surface 2a and 4a. With certain polymeric films it might be advantageous to provide the sample distribution system 6 not only with a hydrophilic coating 17 but also with hydrophobic guidance elements 16 to achieve optimal distribution of the sample.

Main difference to the previously described embodiments for agglutination is the coating of active ingredients and their combination on the first and second surfaces. The AARA requires more than two, in this case four active ingredients plus the chromogenic reporter elements containing enzymes such as horseradish peroxidase and enzyme dye substrates. Here it is important to combine only non active combinations of ingredients on one and the same surface of the detection areas. These non active combinations are a mixture of two or more chemical compounds, which will not react with each other such as two enzyme substrates, whereas an active combination represents a mixture of two reactive compounds such as an enzyme and a suitable enzyme substrate or an recognition element (i.e an antibody) and a suitable analyte (e.g. an antigen). These non active combinations provided on one surface in form of a formulation or an ink will be solubilised in contact with the fluid sample medium and subsequently form an active combination with the compounds of the opposite surface after mixing triggering a chemical or physical reaction.

According to this approach, each sample detection area of the first surface will be coated with a calibration formulations, which contains the analyte or an equivalent molecule, the analyte labelled co-enzyme, plus the compounds required for the photometric evaluation such as a chromogen and the enzyme substrate required by the holo-enzyme. Depending on the calibration strategy the concentration of the calibration compound in the calibration formulation will be varied in n-1 or n-2 of n sample detection area thus at least one sample detection area carries no calibration compound. The sample detection areas on the second surface are coated with the recognition formulation containing the recognition element to recognize the analyte of interest, the apo-enzyme, plus additional enzymes if required for the chromogenic reaction.

**Standard Addition Method for AARA**

As mentioned previously, the role of determining whether an analyte exists in a liquid medium is important to physicians. Equally important is the determination of the concentration of an analyte in a liquid medium. In an embodiment of the present invention, the analyte test element is configured for quantitative determination of the analyte of interest based on the linear standard addition method. For this example it is considered a linear relationship of the analyte over the desired monitoring range of the analyte.

Analogue to the above description of the AARA the analyte test element provides four detection areas on the first surface and the second surface aligned mostly congruent to create the sample distribution system. Three of the sample detection areas ($6'a_1$, $6'a_2$, $6'a_3$), are coated with the calibration formulation 18 whereas the calibration formulation coated on the sample detection areas $6'a_2$, $6'a_3$ contains two different predetermined and known concentrations of the calibration compound 33. In contrary, sample detection area $6'a_1$ is coated with a calibration formulation containing no calibration compound or analyte. Sample detection area 6'c is not coated with the calibration compound and used to evaluate the sample background. The sample detection areas ($6a_1$, $6a_2$, $6a_3$) on the second surface 4a are coated with the recognition formulation 19 providing a predetermined concentration of the recognition element 32, apo-enzyme, and if required further enzymes. Again sample detection area 6c analogue to its opposing partner 6'c provides no recognition formulation or a formulation with no active ingredients respectively recognition element. Table 2 provides an overview of the above described configuration.

**Table 2** Functional components for calibration and recognition formulations required for the apo-enzyme assisted recognition assay.

| Position in sample distribution system | 1st Surface Functional compounds of calibration formulations | 2nd Surface Functional compounds of recognition formulation |
|---|---|---|
| **6'$a_1$ / 6$a_1$** | co-enzyme labelled analyte enzyme substrates, chromogens | recognition element (i.e, specific antibody against analyte), apo-enzyme, additional enzymes if required for optical and photometric detection |
| **6'$a_2$ / 6$a_2$** | Predetermined calibration compound C=1(i.e. analyte or equivalent), co-enzyme labelled analyte enzyme substrates, chromogens | recognition element (i.e. specific antibody against analyte), apo-enzyme, additional enzymes if required for optical and photometric detection |
| **6'$a_3$ / 6$a_3$** | Predetermined calibration compound C=2(i.e. analyte or equivalent), co-enzyme labelled analyte enzyme substrates, chromogens | recognition element (i.e. specific antibody against analyte), apo-enzyme, additional enzymes if required for optical and photometric detection |
| **6'c / 6c** | no coating or inert coating without active ingredients | no coating or inert coating without active ingredients |

After applying the liquid sample medium to the sample distribution system, allowing for the re-solubilisation of the calibration and recognition formulation and for the subsequent reactions therein, the light emitter 20 and detection arrangement 23 of a meter measures a first optical absorbance 25a of the liquid medium located in the sample detection area 6'$a_2$ with the first level of calibration compound as given by Figure 13A. The readout of this detection area 6$a_2$ represents a signal proportional to the combined concentration of the calibration compound in 6'$a_2$ and the concentration of the analyte in liquid medium 15. In parallel, a second optical absorbance 26a is measured of the liquid sample medium 15 located in the detection area 6$a_3$ with a second level of calibration compound in 6'$a_3$ representing a signal proportional to the combined concentration of the calibration compound and the concentration of the analyte. Furthermore, a third optical absorbance 27a is determined of the detection area 6$a_1$ containing no calibration compound in the calibration formulation coated in detection area 6'$a_1$. Thus the liquid sample medium 15 contains only the unknown concentration of analyte 28. According to the theory of linear standard addition method, the processing means of the test system can calculate from the measured absorbencies 25a, 26a, 27a and the known concentrations of the calibration compound in sample detection areas 6'$a_2$, 6'$a_3$, the unknown concentration of the analyte, the coefficients for the calibration equation y=c0+c1x, the correlation coefficient and the standard deviation for the performed analysis by linear regression analysis. The specific concentration of the different components contained in the recognition formulation and the various calibration formulations according to Table 2 have to be optimised by standard laboratory experimentation to achieve optimal performance with the analyte test system. Again, if the relationship between the optical absorbance and the concentration of the analyte shows a non linear characteristic the standard addition method can be modified and adapted to such non linear behaviour by providing more sampled detection areas as shown in Figure 9.

## Preparation method of the analyte test element

The analyte test element of the present invention, which is preferably produced in strip form, can easily be prepared by processes to those of ordinary skill in the arts of printing, punching, and laminating. The design of the analyte test element allows a simple and cost efficient production process, which is preferably but not necessarily of a continuous nature.

In a first step of the preparation method, a pattern of the sample distribution system is formed by creating areas of high and low surface energy on a substrate. In a first embodiment, the areas of high surface energy forming the sample pathways and detection areas on the first and second surfaces are created by applying a hydrophilic formulation on a hydrophobic surface of a substrate. As detailed above, it is also possible to create the areas of high and low surface energy by applying a pattern of hydrophobic "guiding elements" on a hydrophilic surface. In the preferred case the substrate has an intermediate hydrophobic character of a commercially available transparent polymer film, whereby

areas of low and high surface energy of the sample distribution system and sample detection areas are created by printing the hydrophilic pathways underneath or surrounded by the hydrophobic pattern of the hydrophobic guiding elements.

The substrate may be formed of a material like glass, polyvinyl acetate, poly-methylmethacrylate, poly-dimethylsiloxane, polystyrenes, polyesters and polyester resins containing fluorene rings, polycarbonates and polycarbonate-polystyrene graft copolymers, terminal modified polycarbonates, polyolefins, cycloolefins and cycloolefin copolymers, and/or olefin-maleimide copolymers.

The application of a hydrophilic pattern on a hydrophobic substrate and/or the application of hydrophobic "guiding elements" on a hydrophilic substrate or any combination of it can be accomplished by contact and non-contact printing, spraying, immersion, or plasma deposition in particular, useful techniques are flexography, lithograph, gravure, solid ink coating methods, or ink-jet-printing processes.

However, the preferred fabrication method is flexography, which allows high-resolution printing on rotary presses and supports high-speed production. It is an established technology for printing on polymer film substrates and widely used in the packaging industry. The optical detection process shown in Figures 10 and 11 requires transparent and clear ink with low viscosity for the hydrophilic pattern. Low viscous inks are preferred to achieve a thin and even coating of about 2-4 microns. The optical window of the ink needs to be in the wavelength range suitable for the optical detection of the chemical reaction. The requirements for hydrophobic inks, apart from the hydrophobic nature, are less stringent and could be used to decorate the analyte test element with a desired colour as well, thus non transparent inks are preferred for this step. The operation of a four-colour flexography-printing machine is established practice and provides no operational problems. The same holds for lithography device.

Most convenient for the preparation of the analyte test element are solvent based inks, which are available in a large variety from various manufactures. Further, all such available inks could be fine tuned with additional additives and pigments to optimise the required parameters. Many of these inks are based on nitrocellulose ethanol or poly vinyl butyral ethanol mixtures and can be obtained e.g. from Sun Chemicals Inc. (35 Waterview Boulevard, Parsippany, NJ, USA) or Flint Ink Inc. (4600 Arrowhead Drive, Ann Arbor, MI, USA)

Even though solvent based or UV curing inks are applicable to prepare the analyte test element, electron beam (EB) curing inks have some preferred properties. These inks provide highest resistance to mechanical and chemical factors, and contain 100% polymers, optionally with pigments, but no volatile organic solvents and photo initiators, which have proven to affect the stability of sensor chemistry. These positive gains in performance characteristics are derived from the ability of electrons to form cross-linked polymeric films and to penetrate the surface.

Inks used in EB curing make use of the polymerising capability of acrylic monomers and oligomers. Acrylic chemistry has a special significance in modern day inks. (6 J.T. Kunjappu. "The Emergence of Polyacrylates in Ink Chemistry," Ink World, February, 1999, p. 40.) The structure of the simplest acrylic compound, acrylic acid, is shown in the formula (I)

$$CH_2{=}CH{-}COOH \qquad (I)$$

The double bond in the acrylic moiety opens up during interaction with electrons (initiation) and forms a free radical that acts on other monomers forming a chain (propagation) leading to high-molecular-weight polymers. As mentioned before, radiation induced polymerisation requires no external initiator since radiation itself generates free radicals with the result that no initiating species will be left in the coating.

A variety of acrylic monomers are available for EB curing that range from simple acrylates such as 2-phenoxyethyl acrylate and isooctyl acrylate, to pre-polymers like bisphenol A, epoxy acrylate and polyester/polyether acrylates (R. Golden. J. Coatings Technol., 69 (1997), p. 83). This curing technology allows the design of "functional inks" with the focus on the desired chemical and physical properties without the necessity of a solvent and curing chemistry required by other inks, which may complicate the design process.

Generally suitable hydrophobic inks might contain monomers, oligomers, and pre-polymers with hydrophobic functions like isooctyl acrylates, dodecyl acrylates, styrene or silicon derivates, systems with partly fluorinated carbon chains, and additional hydrophobing additives and/or fillers such as hydrophobing agents belonging to the TEGO Phobe Series (TEGO Chemie Service, Essen Germany), hydrophobic pigments such as copper phthalocyans, carbon, graphite, or hydrophobic fillers such as silicon modified fumed silica or PTFE powders and PTFE granulates. Due to the vast variety of additives, pigments, and fillers the above suggested compounds will only have exemplary character.

Inks with hydrophilic functions can be realised from a wide selection of ethanol and water-soluble polymers and polymer mixtures. Useful are polymers and polymer derivatives, copolymers and compounds base on alginate, cellulose and cellulose ester, hydroxyethyl cellulose, gum, acrylic acid, polyvinylalcohol, polyethylene-glycol, polyethylene-oxide, vinylpyrolidone, polystyrene sulfonate, poly(methyl vinyl ether/maleic acid), vinylpyrolidone/trimethylammonium copolymers, and alkyl-phosphocholine derivates. Further optimisation can be achieved with organo-modified silicone acrylates additives, which are a cross-linkable species of organo-modified polysiloxanes, and fluorinated surfactants. A general suitable coating provides a contact angle with water of typically less than 35° and a surface energy of typically more than 50 mN/m.

The second step of the production process comprises the application of the recognition formulations, containing the recognition element and other agents to produce a printable and/or dispensable ink forming a uniform layer with in the sample detection areas.

On all corresponding sample detection areas of the opposing surface calibration formulations containing the appropriated amount of calibration compound required for the quantitative or qualitative detection regime is deposited. Depending on the detection mechanism e.g. agglutination, fluorescence assisted agglutination or AARA additional reporter elements can be added to either the recognition formulation or the calibration formulation as required, details are given above and in Table 1.

Since, the concentration level respectively the total amount of the recognition element applied to the predetermined sample detection areas $6a_1$ to $6a_3$ is responsible for the sensitivity and dynamic range of the various discussed analyte test elements, as well as the concentration level and precision of the applied calibration compound is responsible for the accuracy of the test results, it is paramount for this application to provide analyte test elements with a precise dosage of the above elements, compounds, and ingredients. Such precise dosage can be implemented for example using a micro dispenser system available from Vermes Technik GmbH & Co. KG, Palnkamer Str. 18-20, D-83624 Otterfing, Germany. This system will also allow the dosage of nano- and microparticles as required for certain agglutination reactions. The coating formulations must be prepared to be highly soluble by the liquid sample medium to allow a fast and residue free reconstitution after the introduction of the sample fluid.

The next step comprises the lamination procedure, in which the base and cover layer presenting the first and second surfaces of the sample distribution system are laminated onto a centre layer, thereby defining a distance between the first and second surface of the base and cover layer. The centre layer provides a discontinuity to create a cavity for the sample distribution system in the areas where the sample distribution system is formed on the first and second surface of the base and cover layer. The patterns of high and low surface energy formed on the first and second surface of the base and cover layer must be aligned to be mostly congruent to enable the formation of a functional sample distribution system between the first and second surface.

Precise xy-registration of base and cover layers becomes a critical task for the function of the element, if this registration is not achieved, the sample distribution system will not function properly and/or will have a higher variability with regards to the specified sample volume. Registration tolerances should be within +/- 5% of the width of the hydrophilic pathways to achieve good performance.

Figure 20 shows the top view (left) and cross-section (right) of the analyte test element and the effect of registration quality. In case of 20a the sample distribution system is assembled properly with good alignment of the hydrophilic pathways of the first 2a and second 4a surface. The result of an improperly aligned analyte test element is given in Figure 20b. Although, the spacer between the base (2) and the cover layer (4) is identical in case of 20a and 20b the sample volume is falsely enlarged in case b, since the sample fluid covers partly the hydrophobic guiding elements of the sample distribution system. The effect is caused by the sample fluid inside the analyte test element, which seeks to minimize the surface area exposed to air in order to gain the most favourable energetic state and therefore overriding the effect of the hydrophobic areas.

In an alternative embodiment, as shown in Figure 20c, the sample distribution system of the cover layer 4 is designed about 10% smaller as the sample distribution system of the base layer 2 thus the total sample volume of the analyte test element is defined by the extensions of the sample distribution system of the base layer, allowing a higher tolerance for the registration process during manufacturing without compromising the precision of the required sample volume. It will be obvious for someone skilled in the art that base and cover layer are exchangeable in the discussed embodiment without affecting the principle of the invention.

The application of the centre layer, which may be a double-sided adhesive tape with a preferred thickness of 80 microns or alternatively a hot melt adhesive deposited in an equivalent thickness, is less demanding because of the relatively large discontinuity in the material compared to the size of the hydrophilic pathways. Registration is especially important in continuous production lines where the substrate progresses with several meters up to tens of meters per minute. Substrate expansion and web tension make the registration in x-direction (the direction of the web movement) more difficult than the y-direction perpendicular to the web movement.

A preparation method for flexible polymer films providing an accurate registration of the patterns of first and second surface is illustrated in Figure 21 showing parts of a continuous web production process. In a first production step according to Figure 21a, patterns of the sample distribution system 6 of the base and cover layer are printed on one web substrate 49, which represents the material of the produced analyte test elements. As illustrated in Figure 21, the printed patterns of the sample distribution systems 6 are arranged on the web substrates 49 in such a manner that two sample distribution systems are opposite to each other left and right from a mirror line. Optionally, the sample distribution system can be linked in the areas which form the sample application areas. Thus, the position of the predetermined detection areas 6a, 6'a is fixed relative to each other and remains unaffected by the material expansion and

web tension.

The dotted lines 50 indicate the future cutting lines to segregate the analyte test strips, while the dotted lines 51 indicate the mirror line of the strip artwork and the future fold line of the web substrate.

After printing the fluidic pathways of the analyte test element, the detection areas 6a, 6'a of the sample distribution system are coated with the calibration and recognition formulations. For example, the detection areas 6'a of the lower row of the web substrate 49, which will represent the first surface of the analyte test element, are coated with the calibration formulation containing the reporter element and different levels of the calibration compound; one of the calibration formulation (e. g. positioned in $6'a_1$) does not contain calibration compound and delivers the reading of the liquid sample medium, whereas the detection areas 6a of the upper row of the web substrate 49, which will represent the second surface of the analyte test element, are coated with the recognition formulations containing the recognition element and in the special case of the apo-enzyme assisted recognition assay additional parts of the reporter system as well.

Thereafter, an additional layer is laminated on one of the surfaces, e. g. the surface 2a of the base layer 2, representing the centre layer 52 of the analyte test element as shown in Figure 21b. The centre layer 52 may be formed of double-sided adhesive tape or a hot melt adhesive, which provides breakthroughs 5 exposing the sample distribution systems 6 to create cavities for the sample distribution systems in the analyte test elements after the final assembly step.

The analyte test element of the present invention is then assembled by folding the two sides along the mirror line 51, e.g. with help of a folding iron or other suitable equipment, as illustrated in Figure 21c creating a folded and laminated web 53 as shown in Figure 21d. Subsequently, a press roller can secure a tight connection between the centre layer, base and cover layer.

Finally, the laminated web 53 is cut or punched in to the desired product shape, whereas line 50 projects an exemplary shape of the final analyte test strip onto the web 53 before the segregation process. With the preparation method illustrated in Figure 21 the top part of the substrate can be folded on to the bottom part without the danger of loosing the registration in the x-direction of the web and provides an easier method to get the right registration of the first and second surfaces forming the sample distribution system in comparison to single sheet process.

This invention provides a system for determining the concentration of clinical relevant and biochemical analytes which are only available by laboratory supported immunological or similar affinity analytical methods such as Radio-, Diffusion- or Lateralflow-Immunoassays respectively Enzyme-Linked Immunosorbent Assays. Typically, the analyte is detected in a liquid sample medium such as blood, serum, plasma, saliva, brine, interstitial and/or intracellular fluid. The analyte test element is provided with an integrated calibration and quality control system suitable for dry reagent test strip format with a very small sample volume of about 0.5µL. The production of the inventive analyte test element involves only a small number of uncomplicated production steps enabling an inexpensive production of the element.

## Claims

1. An analyte test element for the qualitative and/or quantitative determination of at least one analyte in a physiological or aqueous sample fluid having a first surface (2a) and a second surface (4a) in a predetermined distance opposite from each other, wherein said both surfaces (2a, 4a) are provided with two substantially equivalent patterns forming areas of high and low surface energy which are aligned mostly congruent, whereby the areas of high surface energy create a sample distribution system (6) with at least two detection areas (6a, 6'a), and wherein at least one of the detection areas (6a, 6'a) of the first and second surfaces (2a, 4a) is provided with at least one non-enzymatic recognition element (32), whereby said at least one recognition element (32) is adapted to undergo an affinity reaction with said at least one analyte.

2. The analyte test element according to claim 1, wherein
n predetermined detection areas (6a) of the first surface (2a) are coated with *n* calibration formulations (18) made up of *m* blank formulations and *n-m* formulations containing different levels of a calibration compound (33), whereby *n* is an integer number larger than 2, *m* is an integer number equal or larger than 1, and *n >m,* and
*n* predetermined detection areas (6'a) of the second surface (4a) are coated with a formulation containing the non-enzymatic recognition element (32).

3. The analyte test element according to claim 2, wherein an additional detection area (6c) is provided with unspecific materials which neither contain the analyte recognition element (32) nor the calibration compound (33) enabling the measurement of background signals.

4. The analyte test element according to claim 2, wherein the calibration compound (33) is identical or substantially equivalent to the analyte.

**5.** The analyte test element according to at least one of the preceding claims, wherein the specific recognition element (s) (32) is/are immobilised on microparticles.

**6.** The analyte test element according to at least one of the preceding claims, wherein the recognition formulation and/or the calibration formulation contain(s) reporter element(s), which mediate the optical detection/determination of the analyte.

**7.** The analyte test element according to claim 6, wherein the reporter element is a fluorescent dye.

**8.** The analyte test element according to claim 7, wherein the fluorescent dye has an affinity to the recognition element.

**9.** The analyte test element according to claim 7, wherein the fluorescent dye has an affinity to the analyte.

**10.** The analyte test element according to claim 6, wherein the reporter element is a fluorescent molecular rotor.

**11.** The analyte test element according to claim 6, wherein the reporter elements comprise an apo-enzyme assisted recognition assay.

**12.** The analyte test element according to claim 11, wherein the apo-enzyme assisted recognition assay comprises a recognition element, an apo-enzyme, an analyte labelled co-enzyme, a substrate required by the holo-enzyme and a chromogen.

**13.** The analyte test element according to at least one of the preceding claims, wherein the recognition element (32) is an antibody or antibody fragments against the analyte.

**14.** The analyte test element according to at least one of the preceding claims, wherein the specific recognition element (32) is a nucleic acid.

**15.** The analyte test element according to at least one of the preceding claims, wherein the specific recognition element (32) is a receptor of the analyte.

**16.** A method for preparing an analyte test element according to any of claims 1 to 15 comprising the steps:

- generating areas of high and low surface energy on a base layer (2) having a first surface (2a), the areas of high surface energy forming a hydrophilic sample distribution system (6) with *n* predetermined detection areas (6'a), whereby *n* is an integer number larger
- generating a corresponding pattern of areas of high and low surface energy on a cover layer (4) having a second surface (4a),
- coating *n* predetermined detection areas (6a) of the first surface (2a) with *n* calibration formulations (18) made up of *m* blank formulations and *n-m* formulations containing different levels of a calibration compound (33), whereby *n* is an integer number larger than *2, m* is an integer number equal or larger than 1, and *n >m,*
- coating *n* predetermined detection areas (6'a) of the second surface (4a) with a recognition formulation (19) containing a non enzymatic recognition element (32), and
- applying the layers of first and second surfaces to the opposite sides of a centre layer (3) having a discontinuity (5) which provides a cavity for the sample distribution system formed by the areas of high surface energy (6, 6') on the first and second surfaces (2a, 4a) of the base and cover layers (2, 4),

whereby the recognition element (32) is adapted to undergo an affinity reaction with an analyte in a physiological or aqueous sample fluid provided to the detection areas (6a, 6'a).

**17.** A method for preparing an analyte test element according to claim 16, wherein said areas of high surface energy (6, 6') are created by applying a hydrophilic formulation on the first and second surfaces (2a, 4a).

**18.** A method for preparing an analyte test element according to one of claims 16 and 17, wherein said areas of low surface energy are created by applying hydrophobic formulation on the first and second surfaces (2a, 4a).

**19.** A method for preparing an analyte test element according to one of claims 17 and 18, wherein said hydrophilic and/or hydrophobic formulation(s) is/are applied on the first and second surfaces by the means of contact and non-contact printing, spraying, immersion, or plasma deposition in particular flexography, lithography, gravure, solid ink coating methods, or ink-jet-printing.

**20.** A method for preparing an analyte test element according to one of claims 16 to 19, wherein said recognition and/or calibration formulation(s) (18, 19) is/are coated on the detection areas (6a, 6'a) of first and second surface by micro-contact printing, micro dispensing, or ink-jet printing.

**21.** A method for preparing an analyte test element according to one of claims 16 to 20, wherein the base layer (2) and the cover layer (4) are formed from one flexible substrate and folded along a longitudinal mirror line (45) to enclose the centre layer (3) in a manner that the hydrophilic patterns (6, 6') forming the sample distribution system with the predetermined detection areas (6'a, 6a) are aligned and registered to be mostly congruent

**22.** An analyte test system for the qualitative and/or quantitative determination of an analyte in a physiological or aqueous sample fluid comprising
an analyte test element according to one of the claims 1 to 15, wherein
*n* predetermined detection areas (6a) of the first surface (2a) are coated with *n* calibration formulations (18) made up of *m* blank formulations and *n-m* formulations containing different levels of a calibration compound (33), whereby *n* is an integer number larger than 2, *m* is an integer number equal or larger than 1, *and n >m,* and
*n* predetermined detection areas (6'a) of the second surface (4a) are coated with a formulation containing the non-enzymatic recognition element (32),
detection means for detecting changes of optical properties of the physiological sample located in *2n* predetermined detection areas and obtaining *n* results from *2n* predetermined detection areas, and
processing means for calculating all calibration coefficients of a polynomial calibration equation available from the *n* measurements and one regression coefficient to validate the quality of the calculated calibration coefficients of the calibration equation.

**23.** An analyte test system for the qualitative and/or quantitative determination of an analyte in a physiological or aqueous sample fluid according to claim 22 comprising a polarisation plane selector.

**24.** An analyte test system for the qualitative and/or quantitative determination of an analyte in a physiological or aqueous sample fluid according to claim 23 wherein said polarisation plane selector is controlled by a processing means.

**25.** An analyte test system for the qualitative and/or quantitative determination of an analyte in a physiological or aqueous sample fluid according to claim 23 or 24 wherein said polarisation plane selector is made of at least one liquid crystal subunit.

**26.** A method for the qualitative and/or quantitative determination of at least one analyte in a physiological or aqueous sample fluid, said method comprising:

applying a physiological sample fluid to an analyte test element according to one of the claims 1 to 15,
connecting the analyte test element to a detection and processing means,
detecting and relating the signals produced in the different detection areas.

## Patentansprüche

**1.** Analyt-Testelement für die qualitative und/oder quantitative Bestimmung mindestens eines Analyten in einer physiologischen oder wässrigen Probenflüssigkeit mit einer ersten Oberfläche (2a) und einer zweiten Oberfläche (4a) in einem vorgegebenen Abstand gegenüber voneinander, wobei die beiden Oberflächen (2a, 4a) mit zwei im Wesentlichen gleichwertigen / äquivalenten Mustern bereitgestellt werden, die Bereiche mit hoher und niedriger Oberflächenenergie ausbilden, die meist kongruent ausgerichtet sind, wobei die Bereiche mit hoher Oberflächenenergie ein Probenverteilungssystem (6) mit mindestens zwei Nachweisbereichen erzeugen (6a, 6'a), und wobei mindestens einer der Nachweisbereiche (6a, 6'a) der ersten und der zweiten Oberfläche (2a, 4a) mit mindestens einem nicht-enzymatischen Erkennungselement (32) bereitgestellt wird, wobei das mindestens eine Erkennungselement (32) angepasst ist, um eine Affinitätsreaktion mit dem mindestens einen Analyten einzugehen.

**2.** Analyt-Testelement gemäß Anspruch 1, wobei
*n* vorgegebene Nachweisbereiche (6a) der ersten Oberfläche (2a) mit *n* Kalibrierungsformulierungen (18), aufgebaut

aus *m* leeren ("blank") Formulierungen und *n-m* Formulierungen, enthaltend unterschiedliche Konzentrationen einer Kalibrierungsverbindung (33), wobei *n* eine ganze Zahl größer als 2 ist, *m* eine ganze Zahl gleich oder größer als 1 ist und $n>m$, beschichtet sind, und
*n* vorgegebene Nachweisbereiche (6'a) der zweiten Oberfläche (4a) mit einer Formulierung, enthaltend das nicht-enzymatische Erkennungselement (32), beschichtet sind.

**3.** Analyt-Testelement gemäß Anspruch 2, wobei ein zusätzlicher Nachweisbereich (6c) mit unspezifischen Materialien, die weder das Analyt-Erkennungselement (32) noch die Kalibrierungsverbindung (33) enthalten, bereitgestellt wird, was die Messung von Hintergrund-Signalen ermöglicht.

**4.** Analyt-Testelement gemäß Anspruch 2, wobei die Kalibrierungsverbindung (33) mit dem Analyten identisch oder im Wesentlichen äquivalent ist.

**5.** Analyt-Testelement gemäß mindestens einem der vorhergehenden Ansprüche, wobei das/die spezifische(n) Erkennungselement(e) (32) auf Mikropartikeln immobilisiert ist/sind.

**6.** Analyt-Testelement gemäß mindestens einem der vorhergehenden Ansprüche, wobei die Erkennungsformulierung und/oder die Kalibrierungsformulierung Reporterelement(e) enthält/enthalten, welche den/die optische(n) Nachweis/Bestimmung des Analyten vermitteln.

**7.** Analyt-Testelement gemäß Anspruch 6, wobei das Reporterelement ein Fluoreszenzfarbstoff ist.

**8.** Analyt-Testelement gemäß Anspruch 7, wobei der Fluoreszenzfarbstoff eine Affinität zu dem Erkennungselement aufweist.

**9.** Analyt-Testelement gemäß Anspruch 7, wobei der Fluoreszenzfarbstoff eine Affinität zu dem Analyten aufweist.

**10.** Analyt-Testelement gemäß Anspruch 6, wobei das Reporterelement ein fluoreszierender molekularer Rotor ist.

**11.** Analyt-Testelement gemäß Anspruch 6, wobei die Reporterelemente einen Apo-Enzym-unterstützten Erkennungsassay umfassen.

**12.** Analyt-Testelement gemäß Anspruch 11, wobei der Apo-Enzym-unterstützte Erkennungsassay ein Erkennungselement, ein Apo-Enzym, ein Analyt-markiertes Co-Enzym, ein Substrat, das vom Holo-Enzym benötigt wird, und ein Chromogen umfasst.

**13.** Analyt-Testelement gemäß mindestens einem der vorhergehenden Ansprüche, wobei das Erkennungselement (32) ein Antikörper oder Antikörperfragmente gegen den Analyten ist.

**14.** Analyt-Testelement gemäß mindestens einem der vorhergehenden Ansprüche, wobei das spezifische Erkennungselement (32) eine Nukleinsäure ist.

**15.** Analyt-Testelement gemäß mindestens einem der vorhergehenden Ansprüche, wobei das spezifische Erkennungselement (32) ein Rezeptor des Analyten ist.

**16.** Verfahren zur Herstellung eines Analyt-Testelements gemäß jedem beliebigen der Ansprüche 1 bis 15, umfassend die Schritte:

- Erzeugen von Bereichen mit hoher und niedriger Oberflächenenergie auf einer Grundschicht (2) mit einer ersten Oberfläche (2a), wobei die Bereiche mit hoher Oberflächenenergie ein hydrophiles Probenverteilungssystem (6) mit *n* vorgegebenen Nachweisbereichen (6'a) ausbilden, wobei *n* eine ganze Zahl größer als 2 ist,
- Erzeugen eines entsprechenden Musters von Bereichen mit hoher und niedriger Oberflächenenergie auf einer Deckschicht (4) mit einer zweiten Oberfläche (4a),
- Beschichten von *n* vorgegebenen Nachweisbereichen (6a) der ersten Oberfläche (2a) mit *n* Kalibrierungsformulierungen (18), aufgebaut aus *m* leeren ("blank") Formulierungen und *n-m* Formulierungen, enthaltend unterschiedliche Konzentrationen einer Kalibrierungsverbindung (33), wobei *n* eine ganze Zahl größer als 2 ist, *m* eine ganze Zahl gleich oder größer als 1 ist und $n>m$,
- Beschichten von *n* vorgegebenen Nachweisbereichen (6'a) der zweiten Oberfläche (4a) mit einer Erkennungsformulierung (19), enthaltend ein nichtenzymatisches Erkennungselement (32), und
- Aufbringen der Schichten der ersten und zweiten Oberflächen auf den gegenüberliegenden Seiten einer Mittelschicht (3) mit einer Diskontinuität (5), welche einen Hohlraum für das Probenverteilungssystem bereitstellt, ausgebildet durch die Bereiche mit hoher Oberflächenenergie (6, 6') auf der ersten und zweiten Oberfläche (2a, 4a) der Grund- und Deckschicht (2, 4),

wobei das Erkennungselement (32) angepasst ist, um eine Affinitätsreaktion mit einem Analyten in einer physiologischen oder wässrigen Probenflüssigkeit, die in den Nachweisbereichen (6a, 6'a) bereitgestellt wird, einzugehen.

**17.** Verfahren zur Herstellung eines Analyt-Testelements gemäß Anspruch 16, wobei die Bereiche mit hoher Oberflächenenergie (6, 6') durch Aufbringen einer hydrophilen Formulierung auf der ersten und zweiten Oberfläche (2a, 4a) erzeugt werden.

**18.** Verfahren zur Herstellung eines Analyt-Testelements gemäß einem der Ansprüche 16 und 17, wobei die Bereiche mit niedriger Oberflächenenergie durch Aufbringen einer hydrophoben Formulierung auf der ersten und zweiten Oberfläche (2a, 4a) erzeugt werden.

**19.** Verfahren zur Herstellung eines Analyt-Testelements gemäß einem der Ansprüche 17 und 18, wobei die hydrophile und/oder hydrophobe Formulierung(en) auf der ersten und zweiten Oberfläche mit Hilfe von Kontakt- und Nichtkontaktdruckverfahren, Sprühen, Eintauchen oder Plasma-Abscheidung, insbesondere Flexographie, Lithographie, Tiefdruck, Festtinte-Beschichtungsverfahren oder Tintenstrahldruck, aufgebracht wird/werden.

**20.** Verfahren zur Herstellung eines Analyt-Testelements gemäß einem der Ansprüche 16 bis 19, wobei die Erkennungs- und/oder Kalibrierungsformulierung(en) (18, 19) auf die Nachweisbereiche (6a, 6'a) der ersten und zweiten Oberfläche durch Mikro-Kontaktdruckverfahren, Mikrodispensierung oder Tintenstrahldruck aufgebracht wird/werden.

**21.** Verfahren zur Herstellung eines Analyt-Testelements gemäß einem der Ansprüche 16 bis 20, wobei die Grundschicht (2) und die Deckschicht (4) aus einem flexiblen Substrat gebildet werden und entlang einer longitudinalen Spiegellinie (45) gefaltet werden, um die Mittelschicht (3) in einer Weise so zu umschließen, dass die hydrophilen Muster (6, 6'), die das Probenverteilungssystem mit den vorgegebenen Nachweisbereichen (6'a, 6a) ausbilden, so ausgerichtet und registriert sind, dass sie meist kongruent sind.

**22.** Analyt-Testsystem für die qualitative und/oder quantitative Bestimmung eines Analyten in einer physiologischen oder wässrigen Probenflüssigkeit, umfassend ein Analyt-Testelement gemäß einem der Ansprüche 1 bis 15, wobei *n* vorgegebene Nachweisbereiche (6a) der ersten Oberfläche (2a) mit *n* Kalibrierungsformulierungen (18), aufgebaut aus *m* leeren ("blank") Formulierungen und *n-m* Formulierungen, enthaltend unterschiedliche Konzentrationen einer Kalibrierungsverbindung (33), wobei *n* eine ganze Zahl größer als 2 ist, *m* eine ganze Zahl gleich oder größer als 1 ist und *n>m,* beschichtet sind, und
*n* vorgegebene Nachweisbereiche (6'a) der zweiten Oberfläche (4a) mit einer Formulierung, enthaltend das nichtenzymatische Erkennungselement (32), beschichtet sind,
Nachweismittel zum Nachweis von Veränderungen der optischen Eigenschaften der physiologischen Probe, die in *2n* vorgegebenen Nachweisbereichen untergebracht sind, und Erhalt von *n* Ergebnissen aus *2n* vorgegebenen Nachweisbereichen, und
Prozessierungsmittel zur Berechnung aller Kalibrierungskoeffizienten einer polynomialen Kalibrierungsgleichung, die aus den *n* Messungen und einem Regressionskoeffizienten erhältlich ist, um die Qualität der berechneten Kalibrierkoeffizienten der Kalibrierungsgleichung zu validieren.

**23.** Analyt-Testsystem für die qualitative und/oder quantitative Bestimmung eines Analyten in einer physiologischen oder wässrigen Probenflüssigkeit gemäß Anspruch 22, umfassend einen Polarisationsebenenselektor.

**24.** Analyt-Testsystem für die qualitative und/oder quantitative Bestimmung eines Analyten in einer physiologischen oder wässrigen Probenflüssigkeit gemäß Anspruch 23, wobei der Polarisationsebenenselektor durch ein Prozessierungsmittel gesteuert wird.

**25.** Analyt-Testsystem für die qualitative und/oder quantitative Bestimmung eines Analyten in einer physiologischen oder wässrigen Probenflüssigkeit gemäß Anspruch 23 oder 24, wobei der Polarisationsebenenselektor aus mindestens einer Flüssigkristall-Untereinheit gebildet wird.

**26.** Verfahren zur qualitativen und/oder quantitative Bestimmung mindestens eines Analyten in einer physiologischen oder wässrigen Probenflüssigkeit, wobei das Verfahren umfasst:

Aufbringen einer physiologischen Probenflüssigkeit auf ein Analyt-Testelement gemäß einem der Ansprüche 1 bis 15,
Anschließen des Analyt-Testelements an ein Nachweis- und Prozessierungsmittel,
Nachweisen und In-Bezug-Setzen der Signale, die in den verschiedenen Nachweisbereichen erzeugt werden.

## Revendications

**1.** Elément de test d'un analyte destiné à déterminer de manière qualitative et/ou quantitative au moins un analyte dans un échantillon de fluide physiologique ou aqueux ayant une première surface (2a) et une seconde surface (4a) se situant à une distance prédéterminée à l'opposé l'une de l'autre, dans lequel lesdites deux surfaces (2a, 4a) sont pourvues de deux zones formant des motifs pratiquement équivalents d'énergies de surface élevée et faible, les zones étant alignées de manière à être quasiment congruentes, moyennant quoi les zones d'énergie de surface élevée créent un système de distribution d'échantillons (6) doté d'au moins deux zones de détection (6a, 6'a), et dans lequel au moins une des zones de détection (6a, 6'a) des première et seconde surfaces (2a, 4a) est dotée d'au moins un élément non enzymatique de reconnaissance (32), moyennant quoi ledit au moins un élément de reconnaissance (32) est adapté pour intervenir dans une réaction d'affinité avec ledit au moins un analyte.

**2.** Elément de test d'un analyte selon la revendication 1, dans lequel
*n* zones de détection prédéterminées (6a) de la première surface (2a) sont enduites avec *n* formulations de calibration (18) composées de *m* formulations à blanc et de *n-m* formulations contenant des teneurs différentes en un composé de calibration (33), moyennant quoi *n* représente un nombre entier supérieur à 2, *m* représente un nombre entier supérieur ou égal à 1, *et n > m,* et
*n* zones de détection prédéterminées (6'a) de la seconde surface (4a) sont enduites avec une formulation contenant l'élément non enzymatique de reconnaissance (32).

**3.** Elément de test d'un analyte selon la revendication 2, dans lequel une zone de détection supplémentaire (6c) est dotée de substances non spécifiques qui ne contiennent ni l'élément de reconnaissance d'un analyte (32) ni le composé de calibration (33) permettant la mesure de signaux du bruit de fond.

**4.** Elément de test d'un analyte selon la revendication 2, dans lequel le composé de calibration (33) est identique ou pratiquement équivalent à l'analyte.

**5.** Elément de test d'un analyte selon au moins une des revendications précédentes, dans lequel le(s) élément(s) de reconnaissance spécifique(s) (32) est/sont immobilisé(s) sur des microparticules.

**6.** Elément de test d'un analyte selon au moins une des revendications précédentes, dans lequel la formulation de l'élément de reconnaissance et/ou la formulation de l'élément de calibration contient(contiennent) un(des) élément (s) rapporteur(s), qui induit(induisent) la détection/détermination optique de l'analyte.

**7.** Elément de test d'un analyte selon la revendication 6, dans lequel l'élément rapporteur est un colorant fluorescent.

**8.** Elément de test d'un analyte selon la revendication 7, dans lequel le colorant fluorescent a une affinité avec l'élément de reconnaissance.

**9.** Elément de test d'un analyte selon la revendication 7, dans lequel le colorant fluorescent a une affinité avec l'analyte.

**10.** Elément de test d'un analyte selon la revendication 6, dans lequel l'élément rapporteur est un rotor moléculaire fluorescent.

**11.** Elément de test d'un analyte selon la revendication 6, dans lequel les éléments rapporteurs comprennent un test de reconnaissance assisté par une apoenzyme.

**12.** Elément de test d'un analyte selon la revendication 11, dans lequel le test de reconnaissance assisté par une apoenzyme comprend un élément de reconnaissance, une apoenzyme, une co-enzyme marquée par un analyte,

un substrat requis par l'holoenzyme et un chromogène.

13. Elément de test d'un analyte selon au moins une des revendications précédentes, dans lequel l'élément de reconnaissance (32) est un anticorps ou des fragments d'anticorps dirigés contre l'analyte.

14. Elément de test d'un analyte selon au moins une des revendications précédentes, dans lequel l'élément de reconnaissance spécifique (32) est un acide nucléique.

15. Elément de test d'un analyte selon au moins une des revendications précédentes, dans lequel l'élément de reconnaissance spécifique (32) est un récepteur de l'analyte.

16. Procédé de préparation d'un élément de test d'un analyte selon l'une quelconque des revendications 1 à 15 comprenant les étapes consistant à :

- générer des zones d'énergies de surface élevée et faible sur une couche de base (2) ayant une première surface (2a), les zones d'énergie de surface élevée formant un système de distribution d'échantillons hydrophiles (6) doté de *n* zones de détection prédéterminées (6'a), moyennant quoi *n* représente un nombre entier supérieur à 2,
- générer un motif équivalent de zones dotées d'énergies de surface élevée et faible sur une couche de couverture (4) ayant une seconde surface (4a),
- *enduire n* zones de détection prédéterminées (6a) de la première surface (2a) avec *n* formulations de calibration (18) composées de *m* formulations à blanc et de *n-m* formulations contenant des teneurs différentes en un composé de calibration (33), moyennant quoi *n* représente un nombre entier supérieur à 2, *m* représente un nombre entier supérieur ou égal à 1, et $n > m$,
- enduire *n* zones de détection prédéterminées (6'a) de la seconde surface (4a) avec une formulation de reconnaissance (19) contenant un élément non enzymatique de reconnaissance (32), et
- appliquer les couches de la première et de la seconde surface des côtés opposés d'une couche centrale (3) ayant une discontinuité (5) fournissant une cavité pour le système de distribution de l'échantillon formé par les zones d'énergie de surface élevée (6, 6') sur la première et la seconde surface (2a, 4a) sur la couche de base et les couches de couverture (2, 4),

moyennant quoi l'élément de reconnaissance (32) est adapté pour intervenir dans une réaction d'affinité avec un analyte dans un échantillon de fluide physiologique ou aqueux déposé sur les zones de détection (6a, 6'a).

17. Procédé de préparation d'un élément de test d'un analyte selon la revendication 16, dans lequel lesdites zones d'énergie de surface élevée (6, 6') sont créées en appliquant une formulation hydrophile sur la première et la seconde surface (2a, 4a).

18. Procédé de préparation d'un élément de test d'un analyte selon l'une des revendications 16 et 17, dans lequel lesdites zones d'énergie de surface faible sont créées en appliquant une formulation hydrophobe sur la première et la seconde surface (2a, 4a).

19. Procédé de préparation d'un élément de test d'un analyte selon l'une des revendications 17 et 18, dans lequel ladite (lesdites) formulation(s) hydrophile(s) et/ou hydrophobe(s) est/sont appliquée(s) sur la première et la seconde surface au moyen d'une impression par contact et par jet d'encre, une application par pulvérisation, une immersion, ou un dépôt par projection plasma, en particulier la flexographie, la lithographie, la gravure, des procédés de revêtement à encre solide, ou d'impression par jet d'encre.

20. Procédé de préparation d'un élément de test d'un analyte selon l'une des revendications 16 à 19, dans lequel ladite (lesdites) formulation(s) de reconnaissance et/ou de calibration (18, 19) est/sont enduite(s) sur les zones de détection (6a, 6'a) de la première et de la seconde surface par impression par microcontact, distribution par micropipette, ou impression par jet d'encre.

21. Procédé de préparation d'un élément de test d'un analyte selon l'une des revendications 16 à 20, dans lequel la couche de base (2) et la couche de couverture (4) sont composées d'un substrat flexible et sont pliées le long d'une ligne miroir longitudinale (45) pour renfermer la couche centrale (3) de manière à ce que les motifs hydrophiles (6, 6') formant le système de distribution de l'échantillon avec les zones de détection prédéterminées (6'a, 6a) soient alignés et fixés de manière à être quasiment congruents.

**22.** Système de test d'un analyte destiné à déterminer de manière qualitative et/ou quantitative un analyte dans un échantillon de fluide physiologique ou aqueux comprenant :

un élément de test d'un analyte selon l'une des revendications 1 à 15, dans lequel *n* zones de détection prédéterminées (6a) de la première surface (2a) sont enduites avec *n* formulations de calibration (18) composées de *m* formulations à blanc et de *n-m* formulations contenant des teneurs différentes en un composé de calibration (33), moyennant quoi *n* représente un nombre entier supérieur à 2, *m* représente un nombre entier supérieur ou égal à 1, et $n > m$, et

*n* zones de détection prédéterminées (6'a) de la seconde surface (4a) sont enduites avec une formulation contenant l'élément non enzymatique de reconnaissance (32),

un moyen de détection destiné à détecter des changements dans les propriétés optiques de l'échantillon physiologique situé dans les *2n* zones de détection prédéterminées et à obtenir *n* résultats des *2n* zones de détection prédéterminées, et

un moyen de traitement destiné à calculer l'ensemble des coefficients de calibration d'une équation de calibration polynomiale disponible à partir des *n* mesures et un coefficient de régression destiné à valider la qualité des coefficients de calibration calculés de l'équation de calibration.

**23.** Système de test d'un analyte destiné à déterminer de manière qualitative et/ou quantitative un analyte dans un échantillon de fluide physiologique ou aqueux selon la revendication 22 comprenant un sélecteur d'un plan de polarisation.

**24.** Système de test d'un analyte destiné à déterminer de manière qualitative et/ou quantitative un analyte dans un échantillon de fluide physiologique ou aqueux selon la revendication 23 dans lequel ledit sélecteur d'un plan de polarisation est commandé par un moyen de traitement.

**25.** Système de test d'un analyte destiné à déterminer de manière qualitative et/ou quantitative un analyte dans un échantillon de fluide physiologique ou aqueux selon la revendication 23 ou 24 dans lequel ledit sélecteur d'un plan de polarisation est composé d'au moins une sous-unité à cristaux liquides.

**26.** Procédé destiné à déterminer de manière qualitative et/ou quantitative au moins un analyte dans un échantillon de fluide physiologique ou aqueux, ledit procédé comprenant les étapes consistant à :

appliquer un échantillon de fluide physiologique sur un élément de test d'un analyte selon l'une des revendications 1 à 15,

connecter l'élément de test d'un analyte à un moyen de détection et de traitement,

détecter et relier les signaux produits dans les différentes zones de détection.

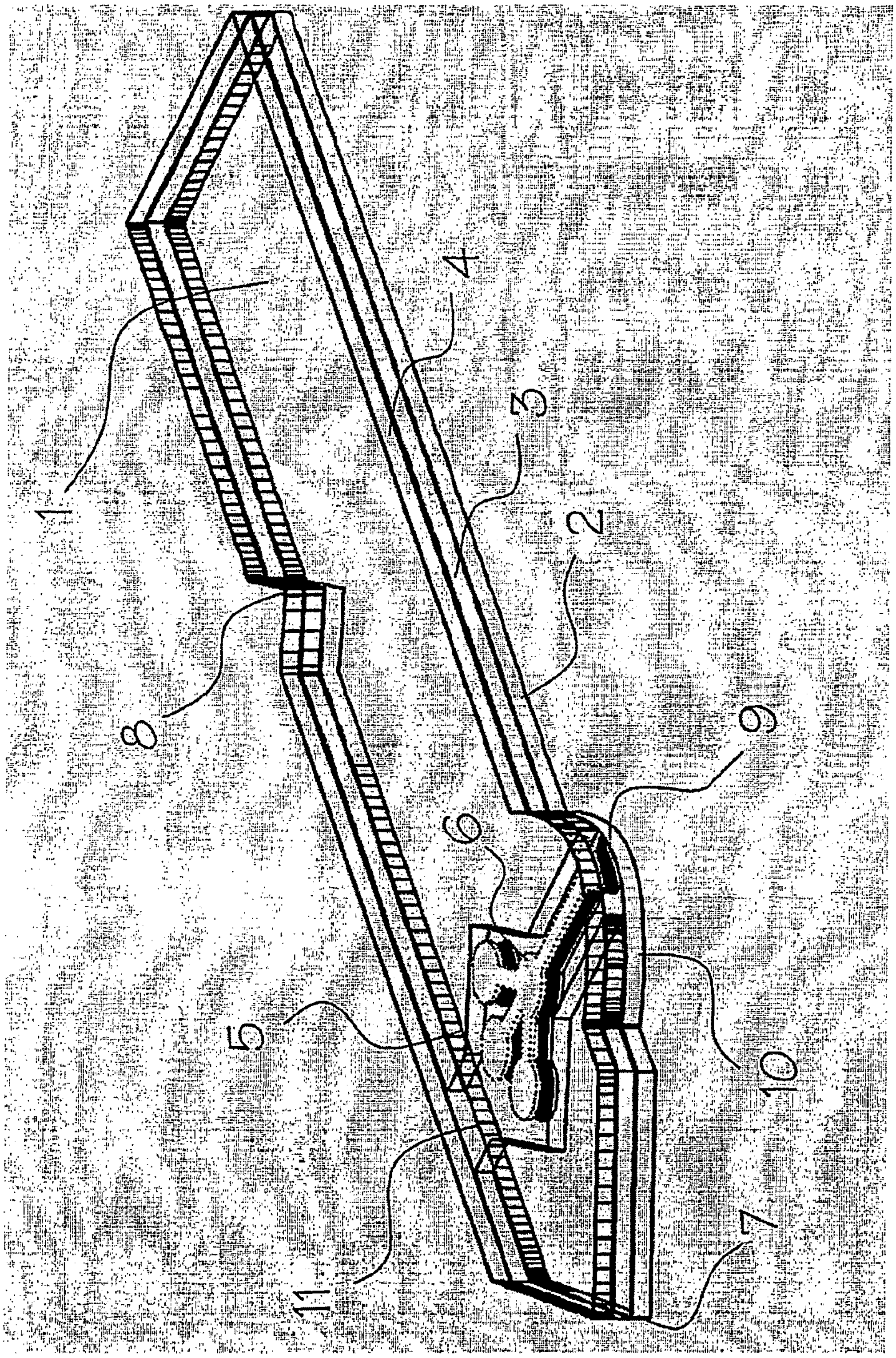


Fig. 1

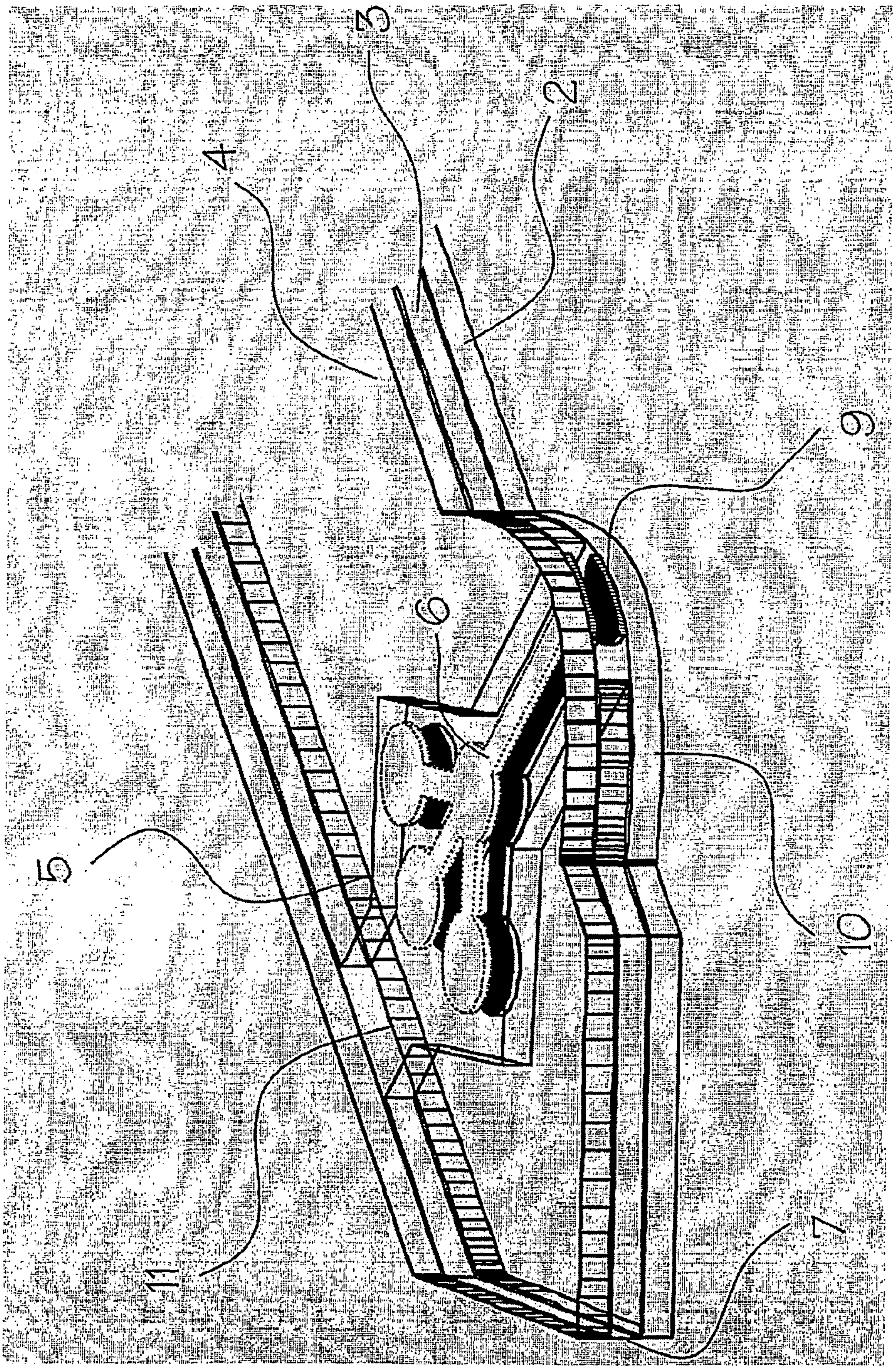


Fig. 2

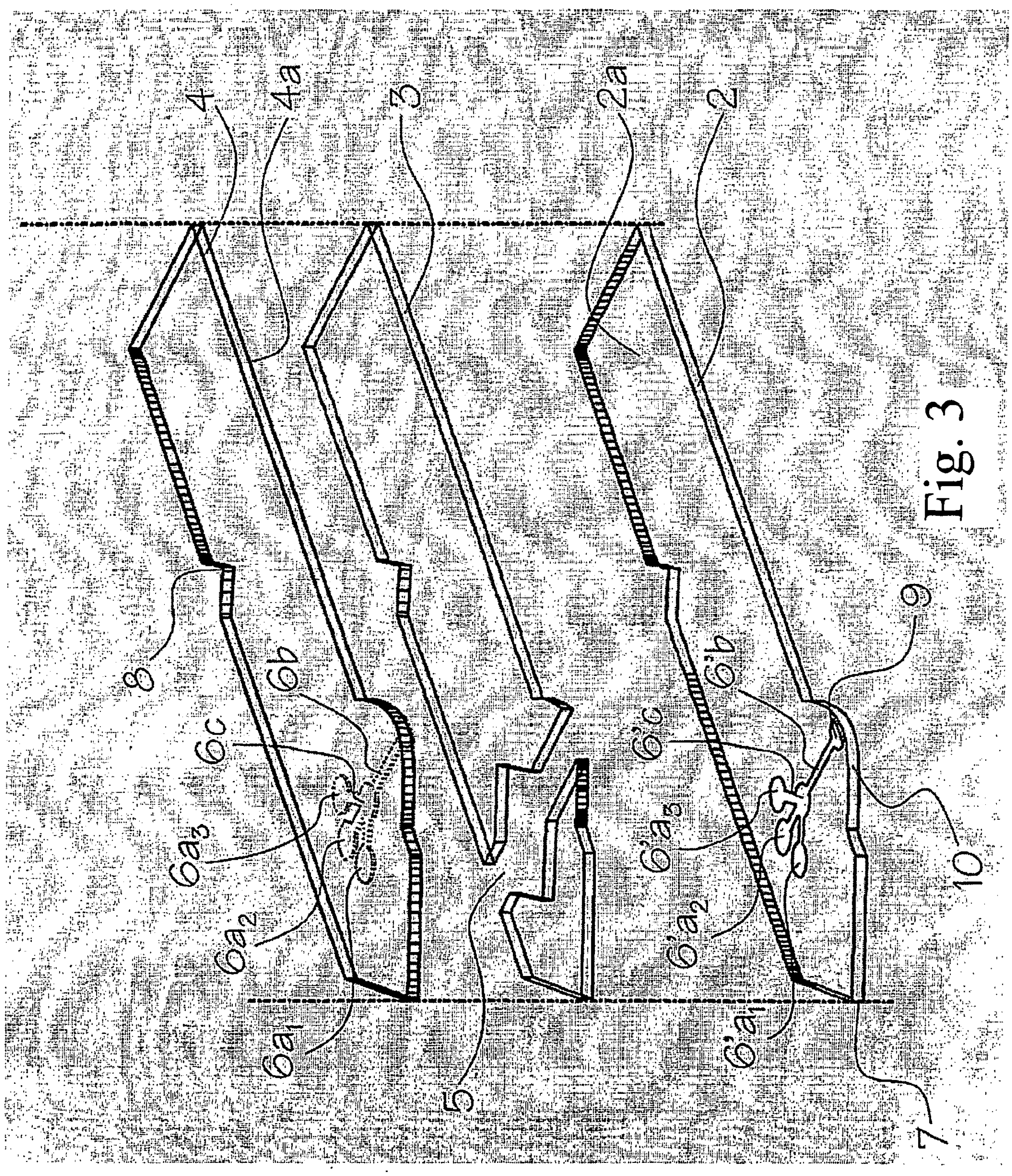


Fig. 3

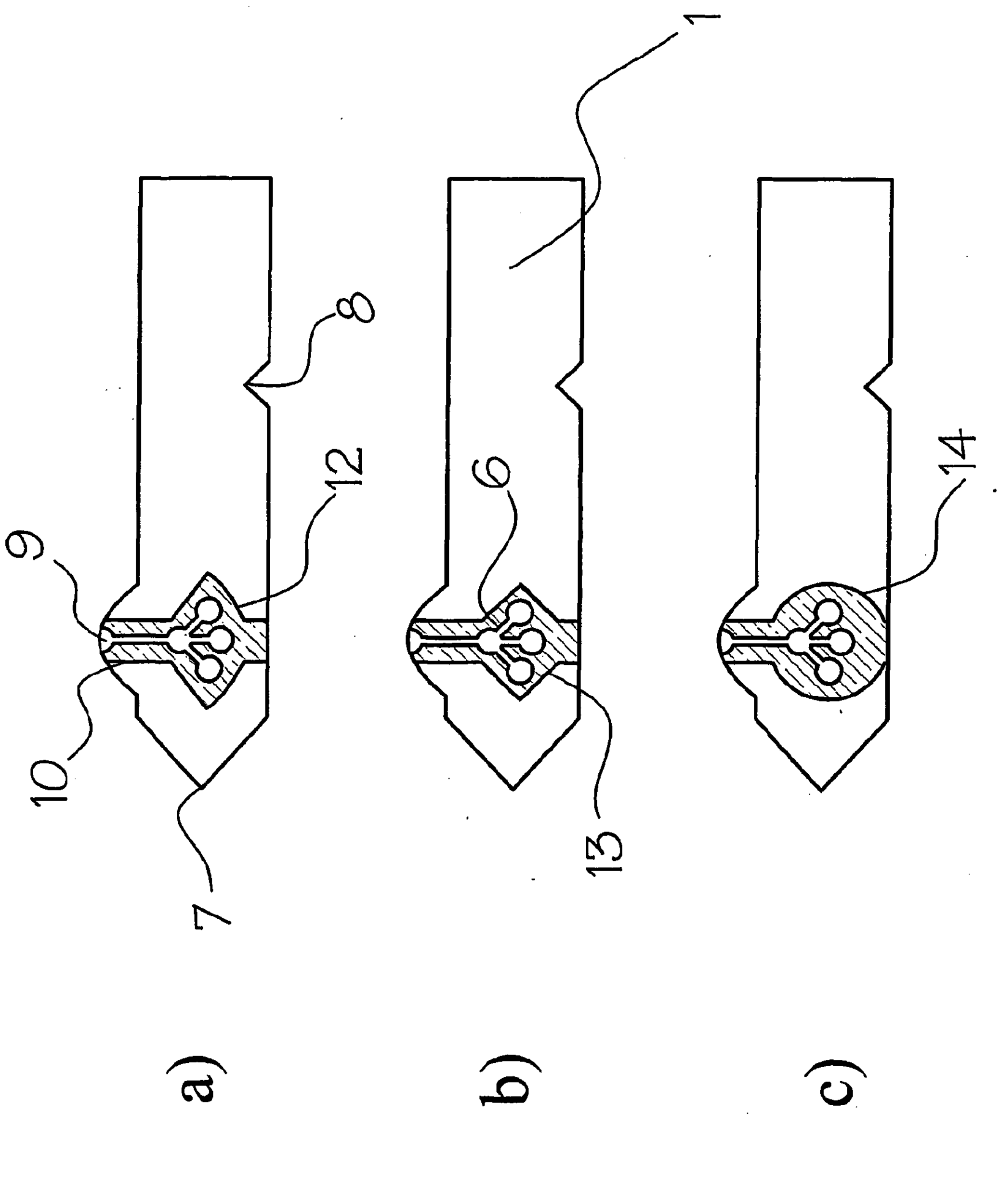


Fig. 4

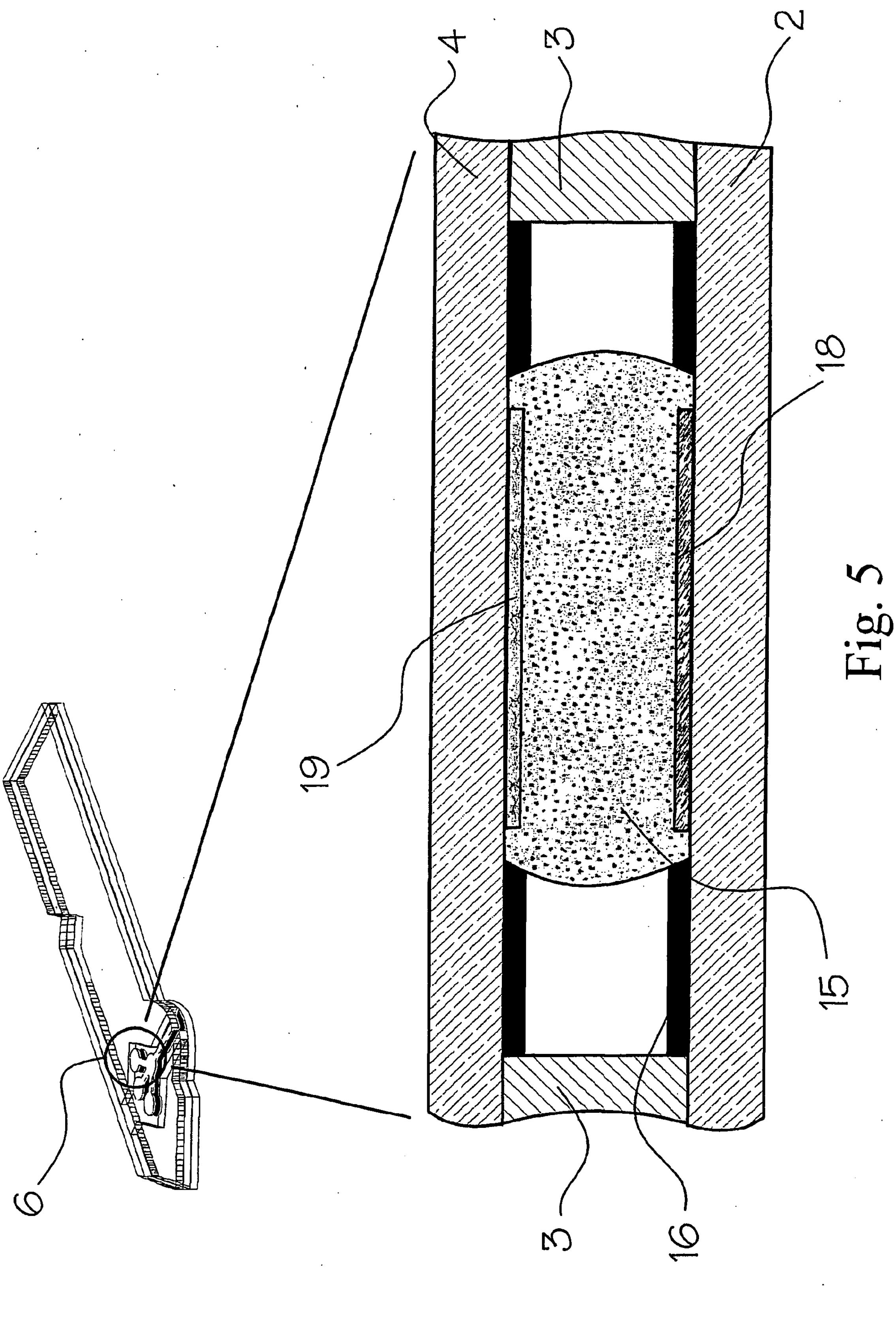


Fig. 5

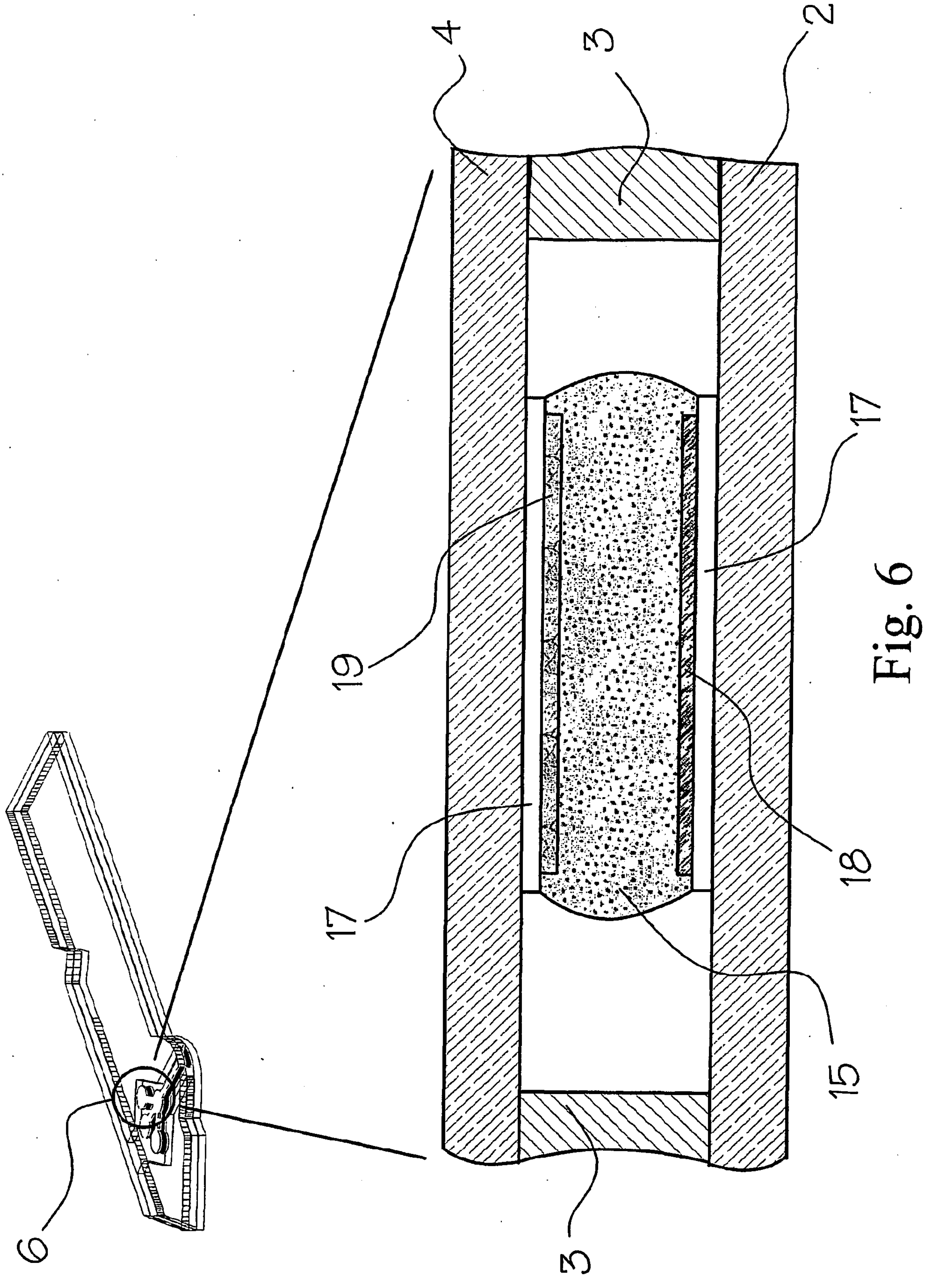


Fig. 6

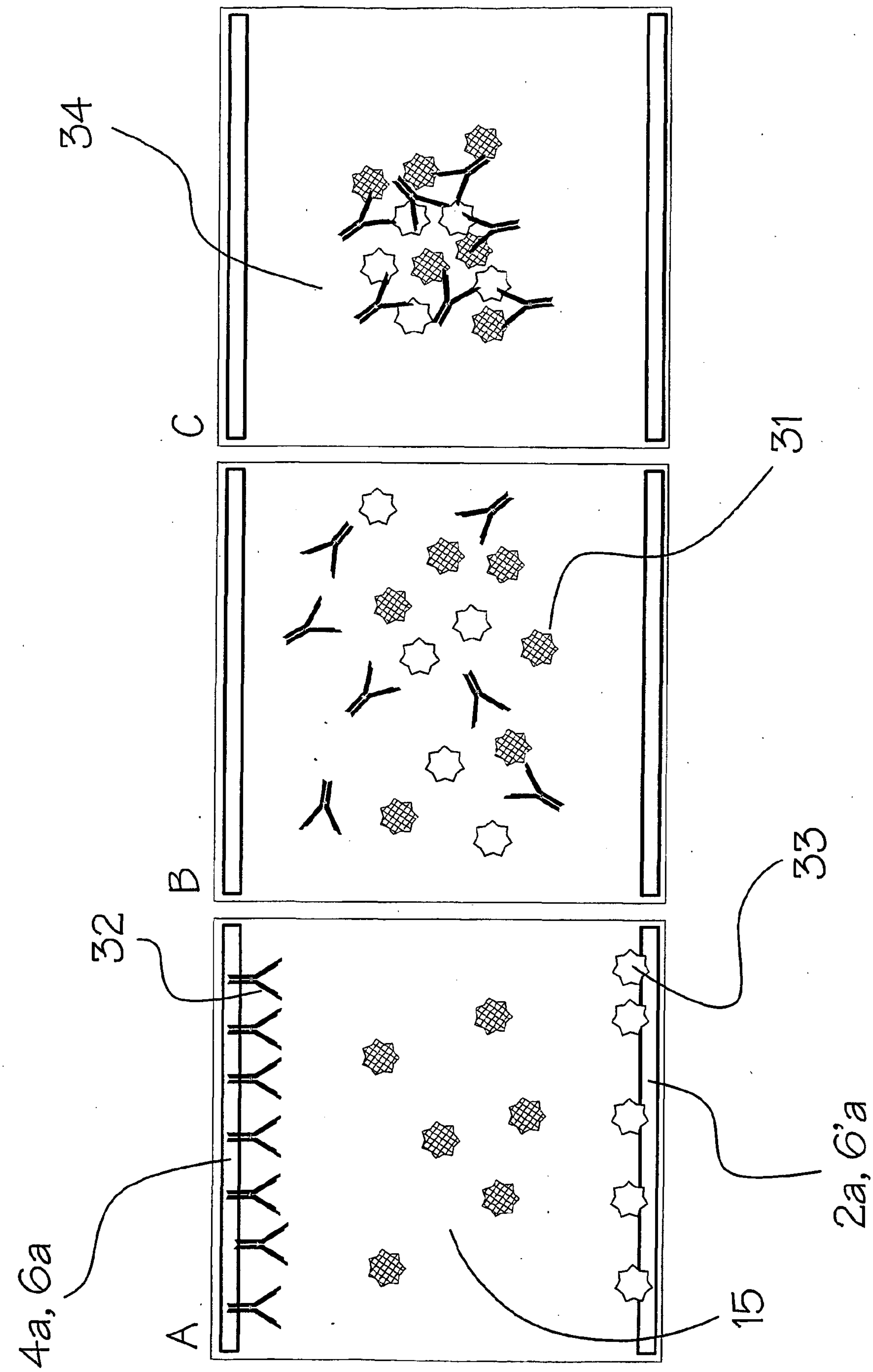


Fig. 7

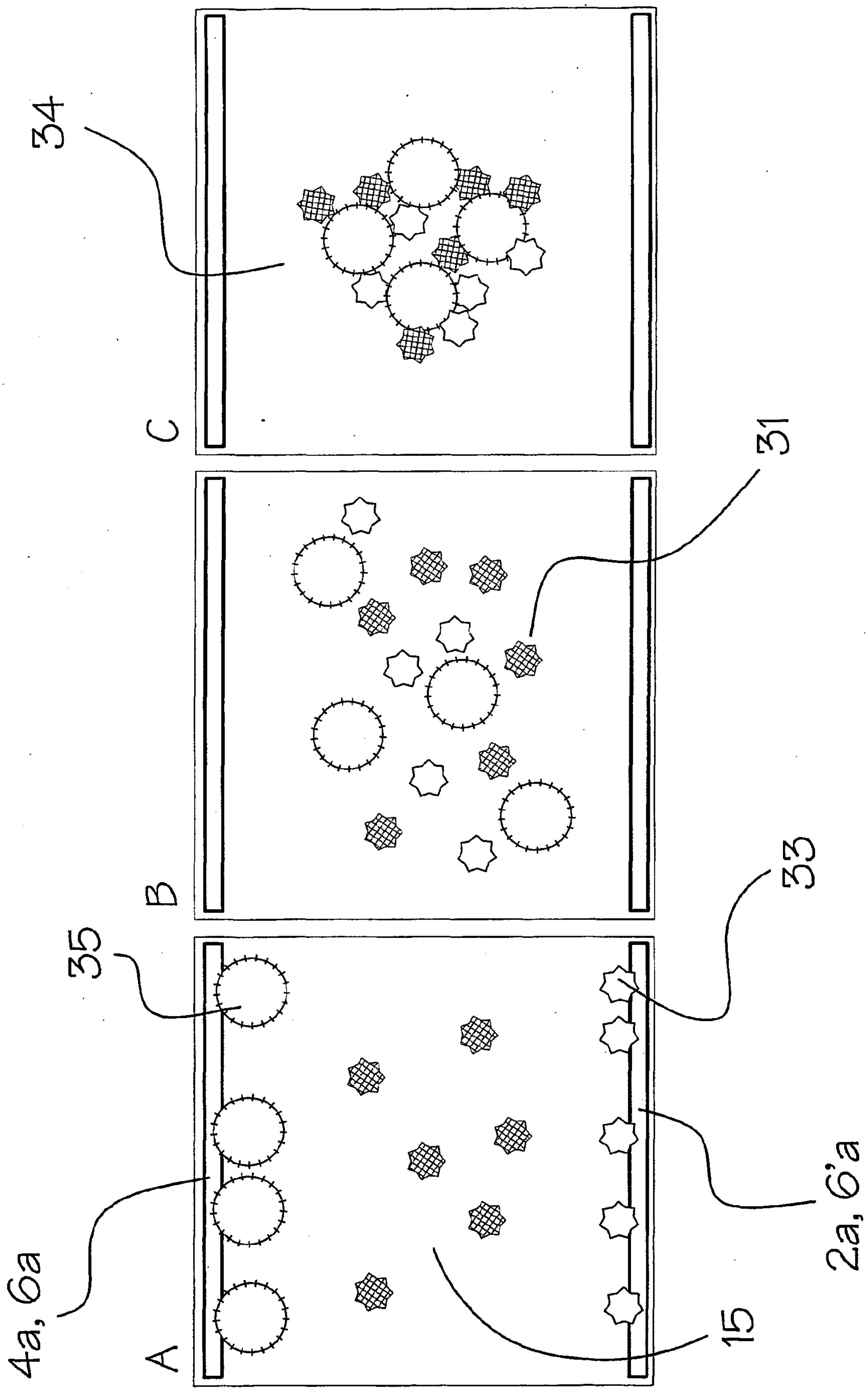


Fig. 8

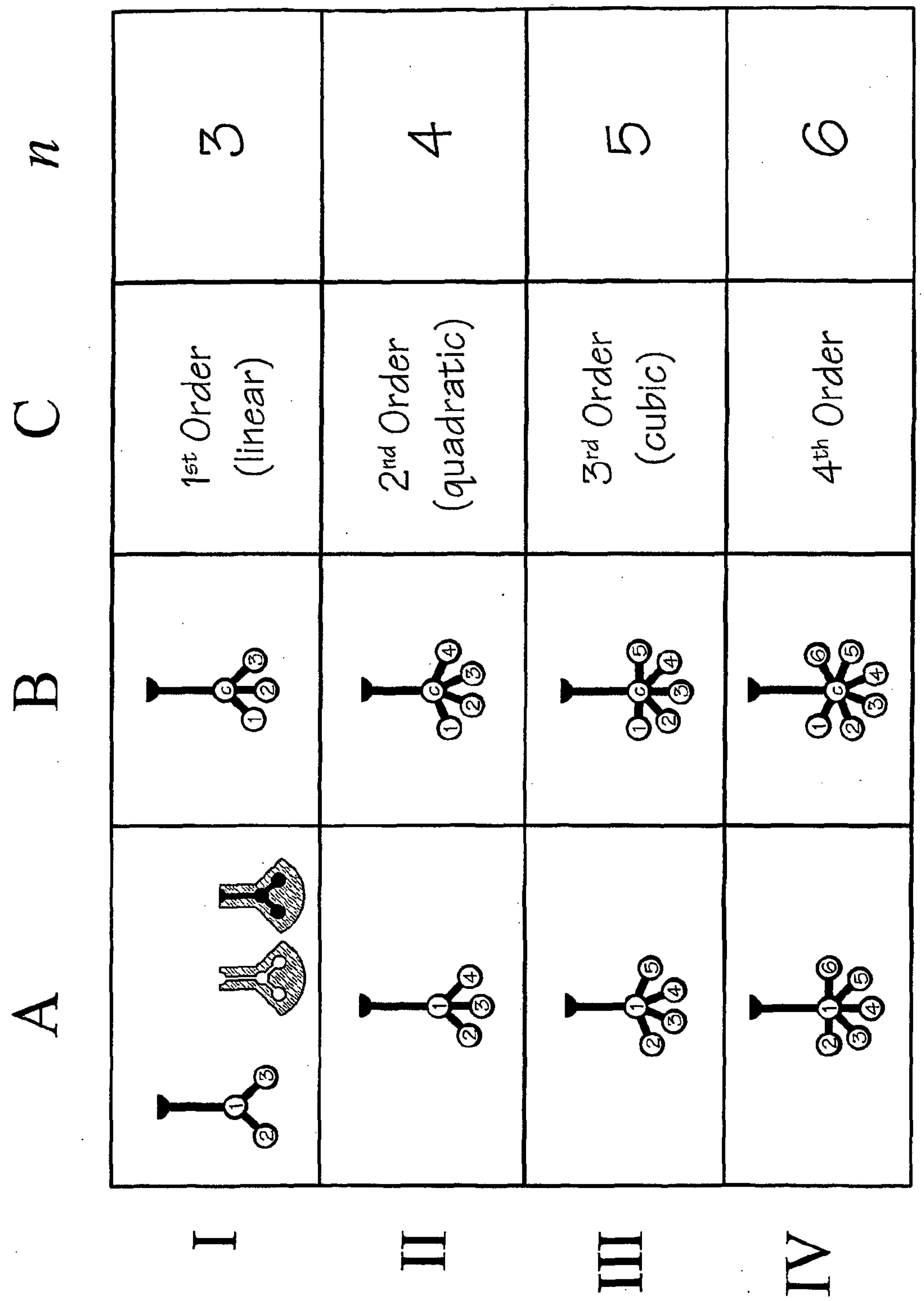
A
B
C
n
I
II
III
IV
1st Order (linear)
2nd Order (quadratic)
3rd Order (cubic)
4th Order
3
4
5
6

Fig. 9

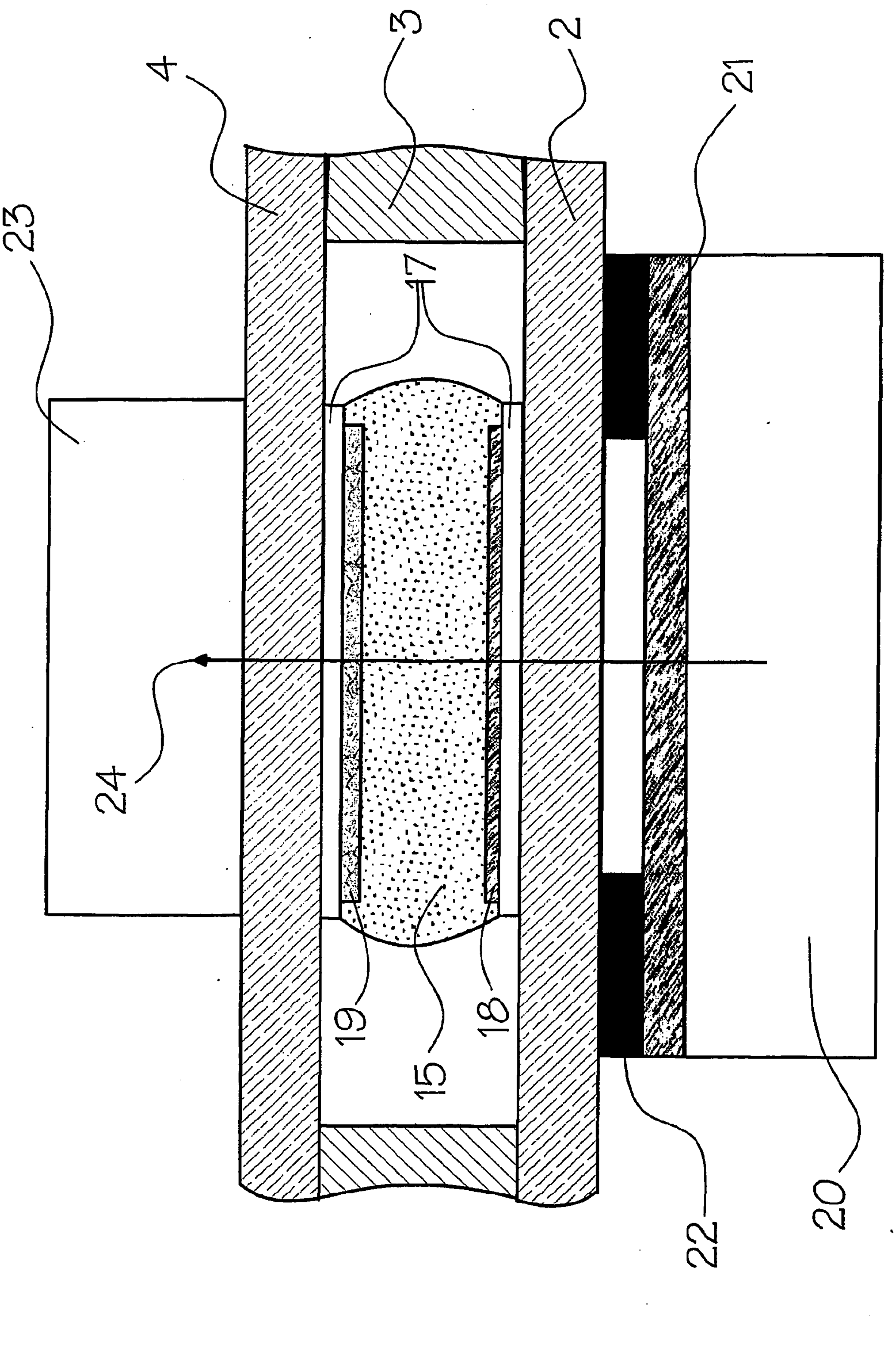


Fig. 10

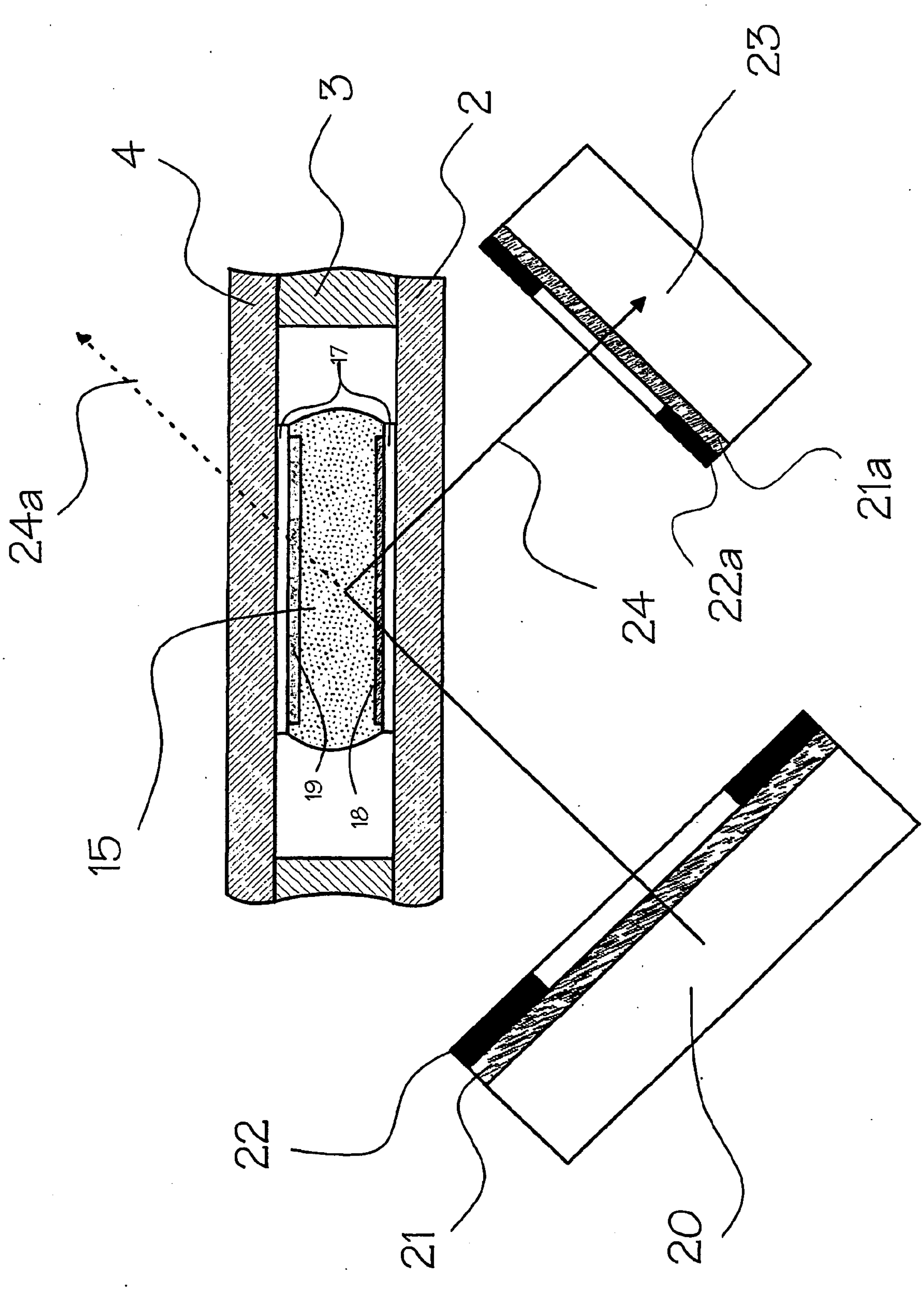


Fig. 11

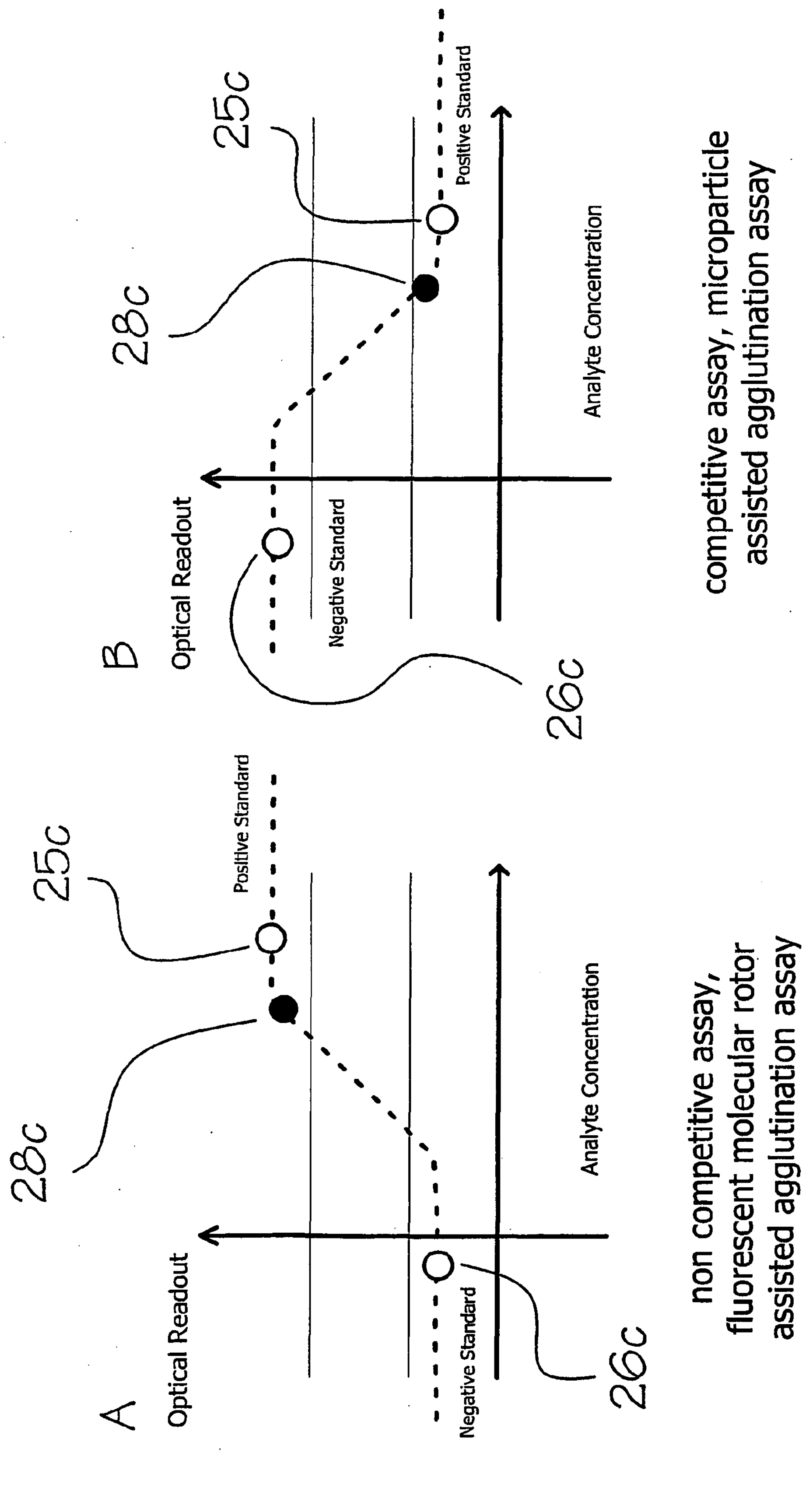
A
Optical Readout
Negative Standard
Positive Standard
Analyte Concentration
25c
28c
26c
non competitive assay,
fluorescent molecular rotor
assisted agglutination assay
B
Optical Readout
Negative Standard
Positive Standard
Analyte Concentration
25c
28c
26c
competitive assay, microparticle
assisted agglutination assay

Fig. 12

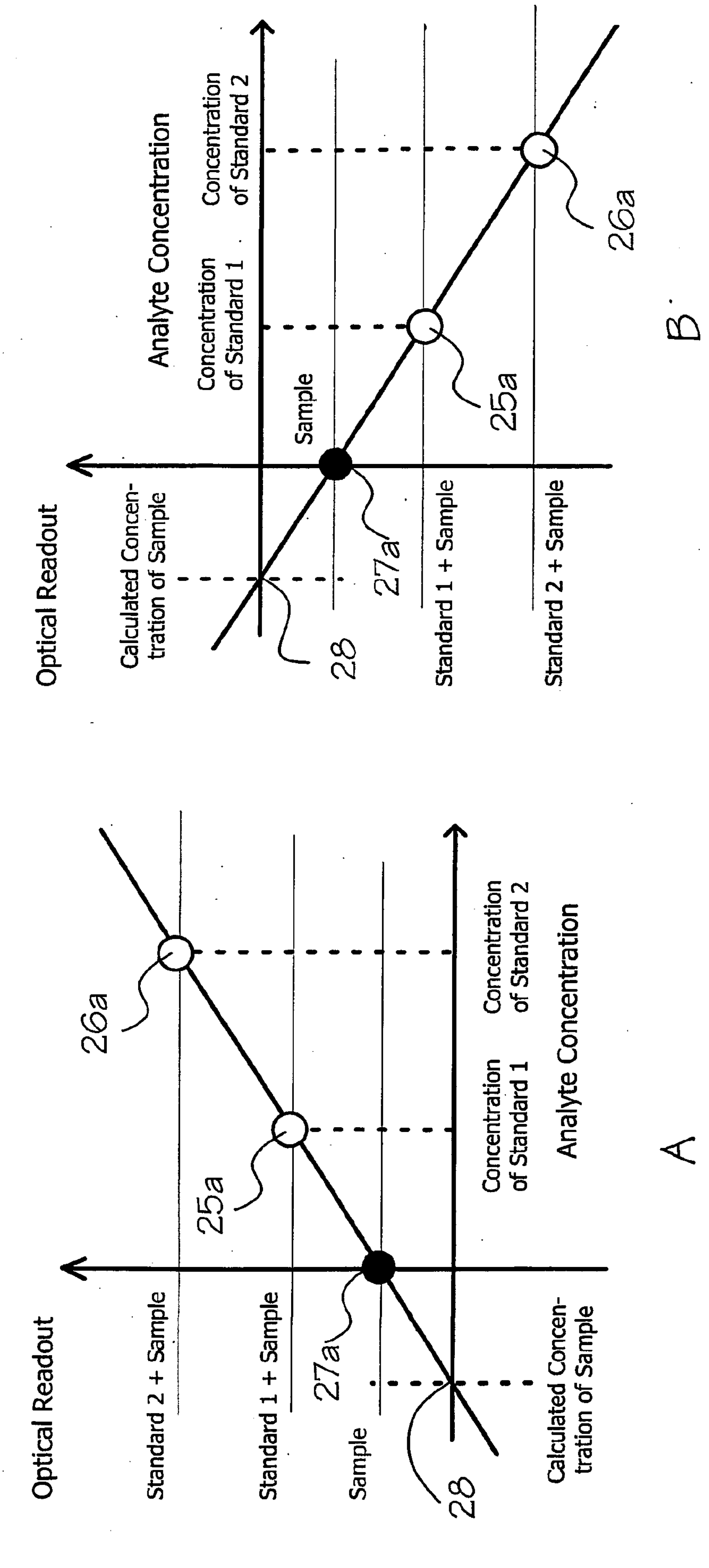
Optical Readout
Standard 2 + Sample
Standard 1 + Sample
Sample
Calculated Concen-
tration of Sample
28
27a
25a
26a
Concentration
of Standard 1
Concentration
of Standard 2
Analyte Concentration
A
Optical Readout
Calculated Concen-
tration of Sample
28
27a
Standard 1 + Sample
Standard 2 + Sample
Sample
25a
26a
Concentration
of Standard 1
Concentration
of Standard 2
Analyte Concentration
B

Fig. 13

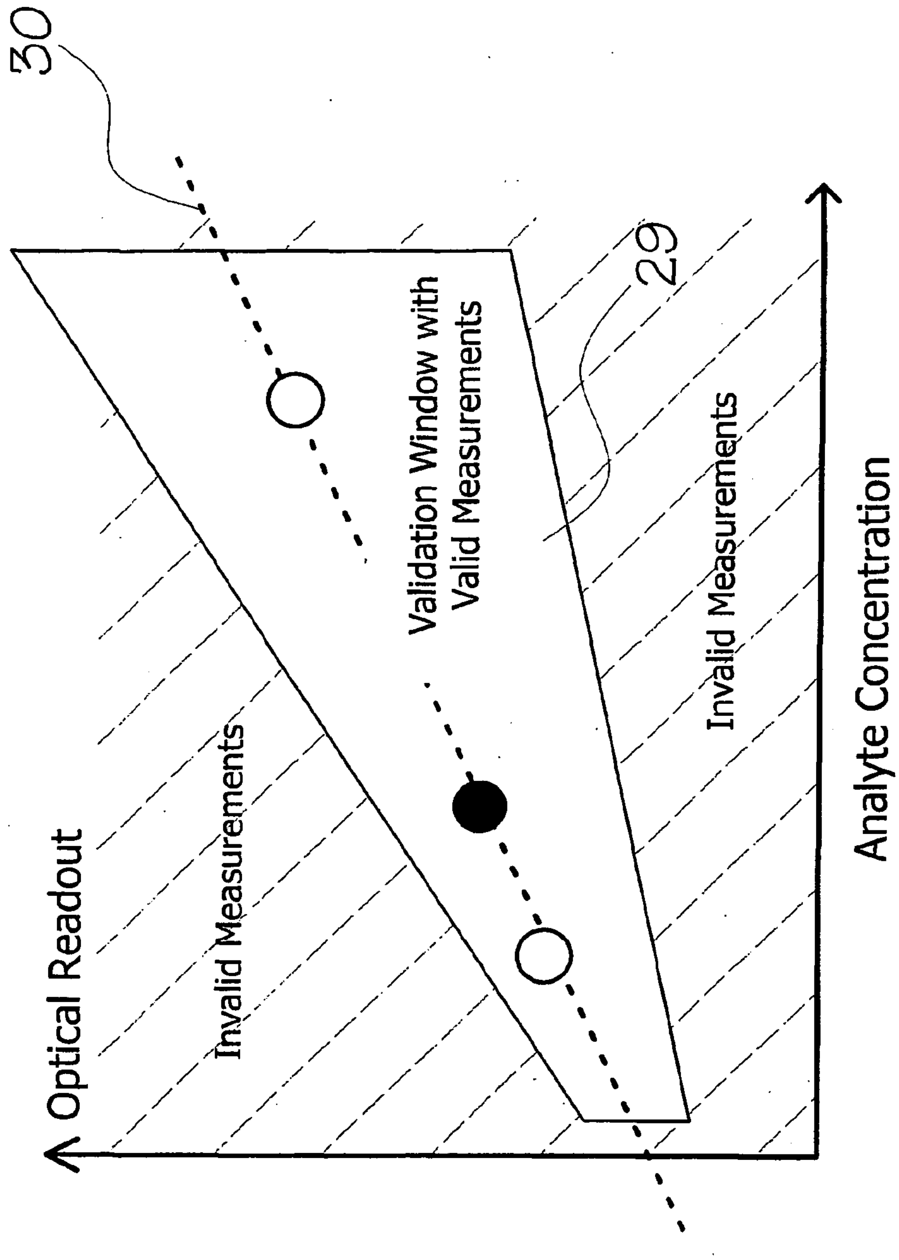
30
29
Optical Readout
Invalid Measurements
Validation Window with
Valid Measurements
Invalid Measurements
Analyte Concentration

Fig. 14

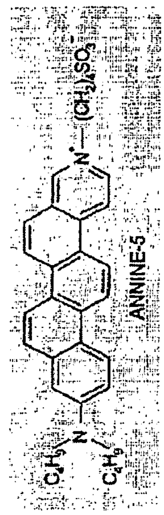
ANNINE-5

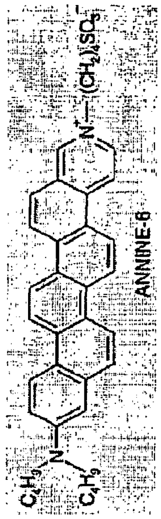
ANNINE-6

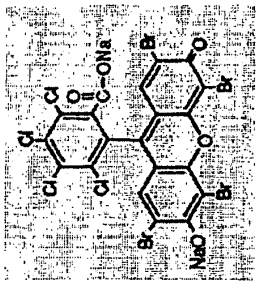
Phloxine B (Magdala Red)

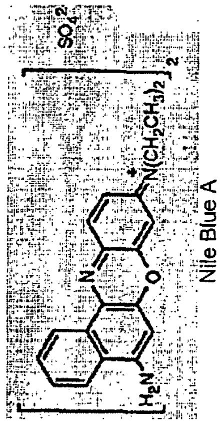
Nile Blue A

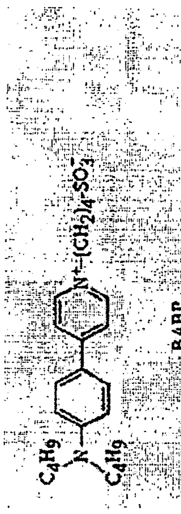
BABP

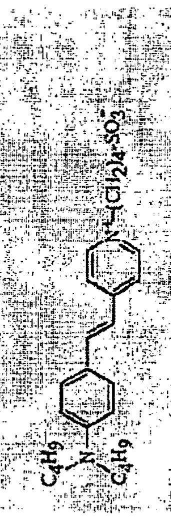
RH 364

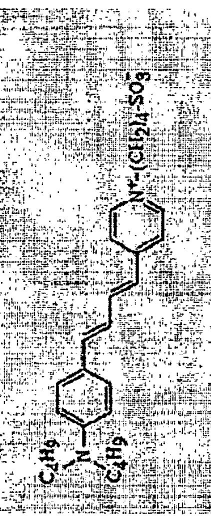
RH 160

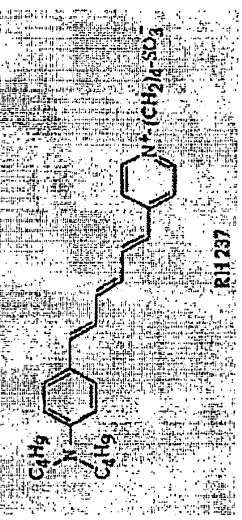
RH 237

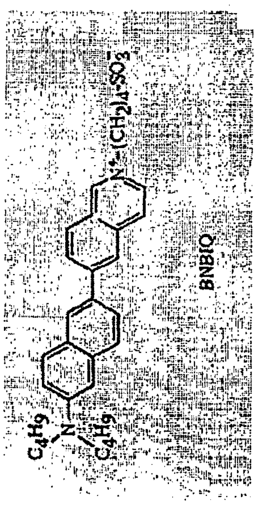
BNBIQ

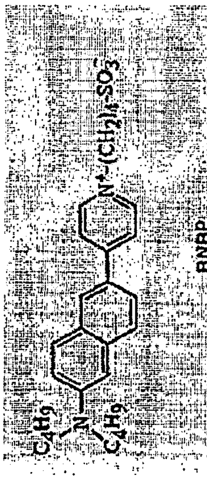
BNBP

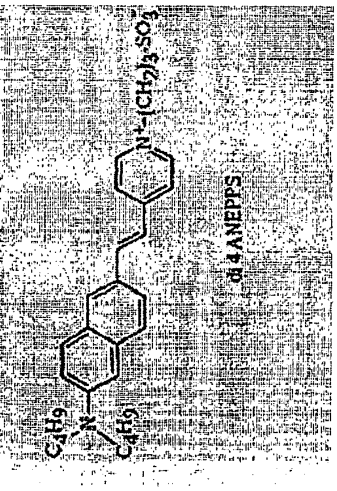
di 4 ANEPPS

Fig. 15

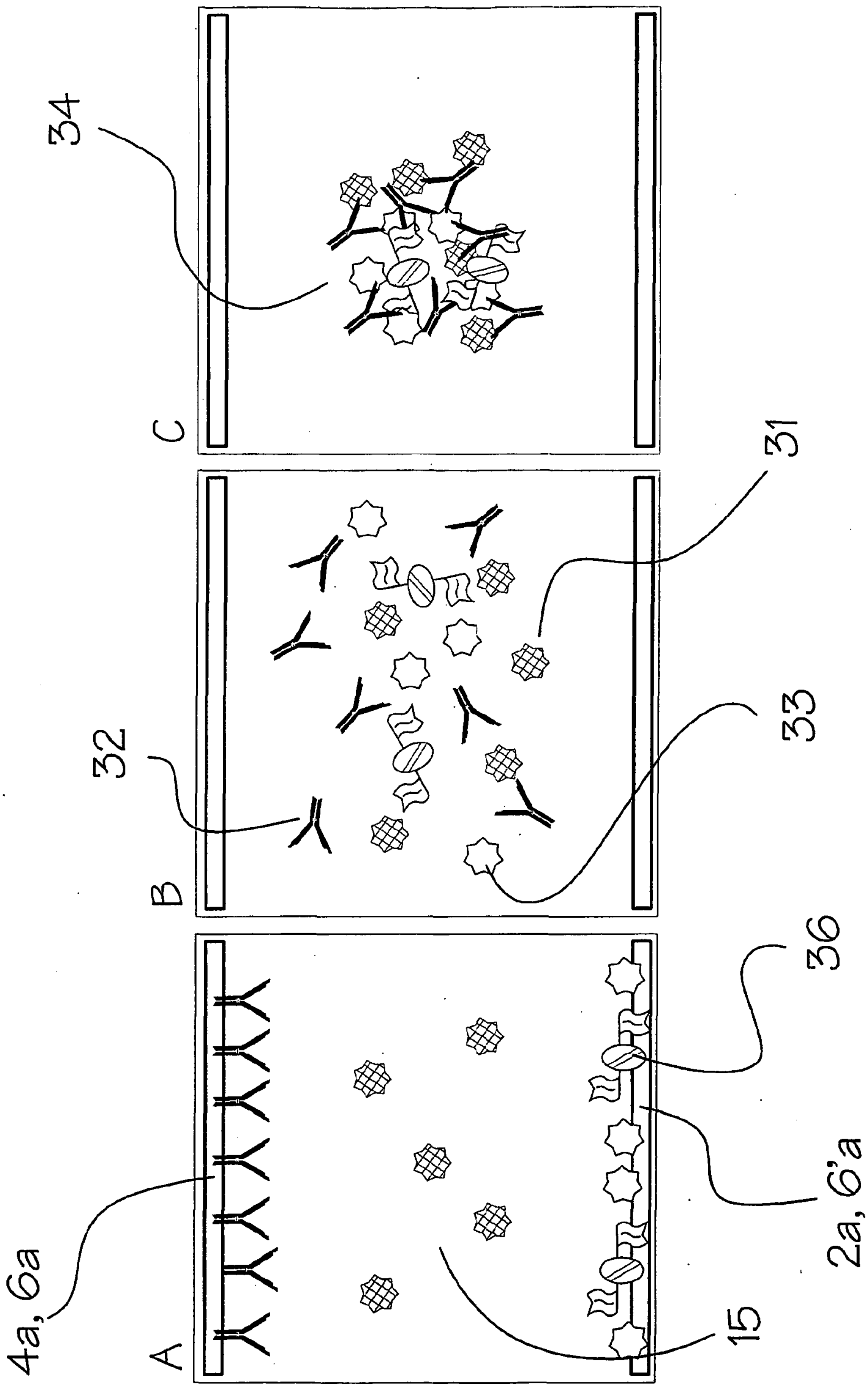


Fig. 16

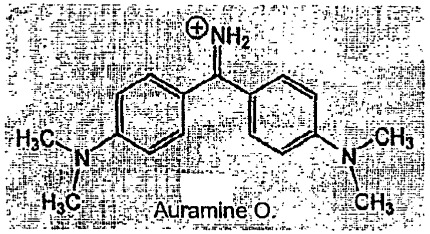

Auramine O

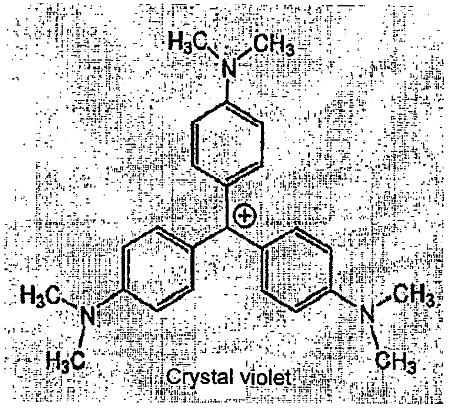

Crystal violet

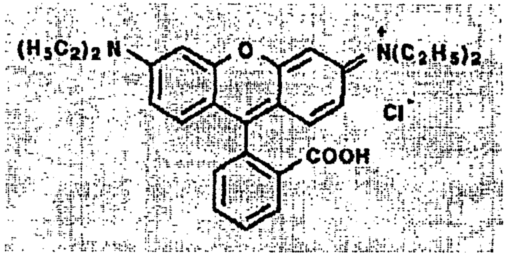

Rhodamine B

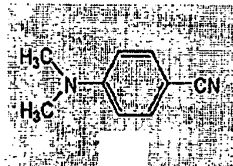

p-N,N-dimethylamino-benzonitrile (DMABN)

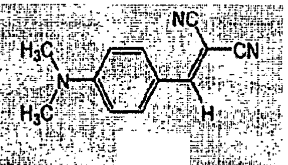

p-N,N-dimethylamino-benzylidenemalononitrile

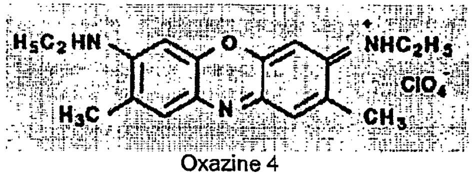

Oxazine 4

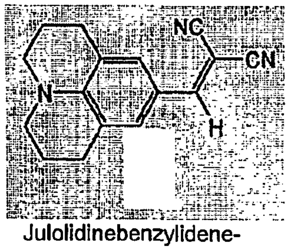

Julolidinebenzylidene-malononitrile

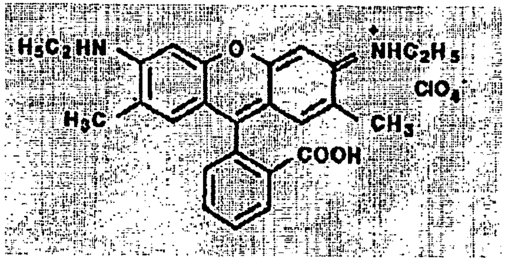

Rhodamine 19

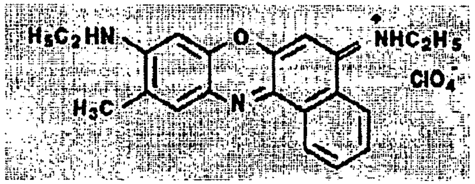

Oxazine 170

Fig. 17

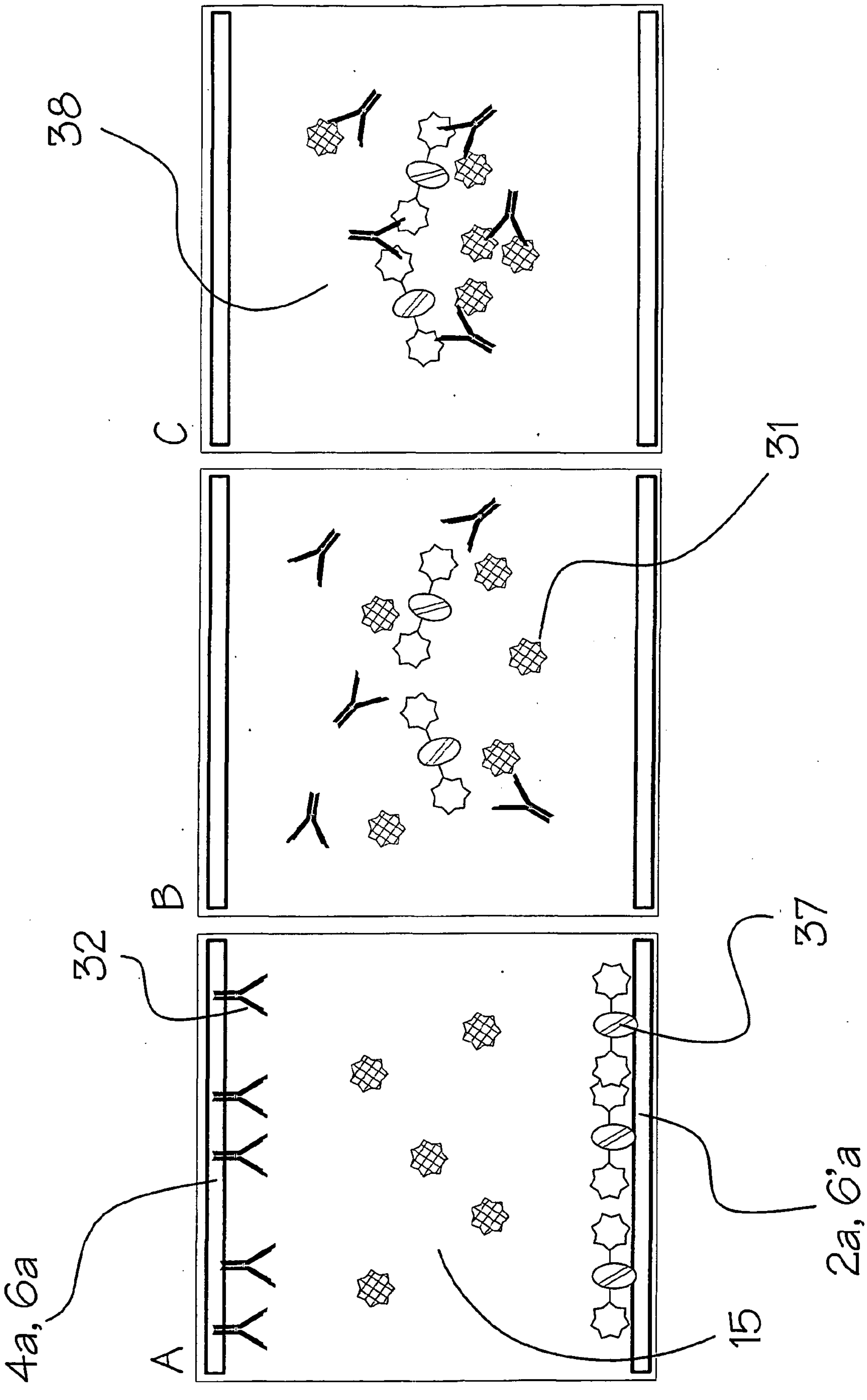


Fig. 18

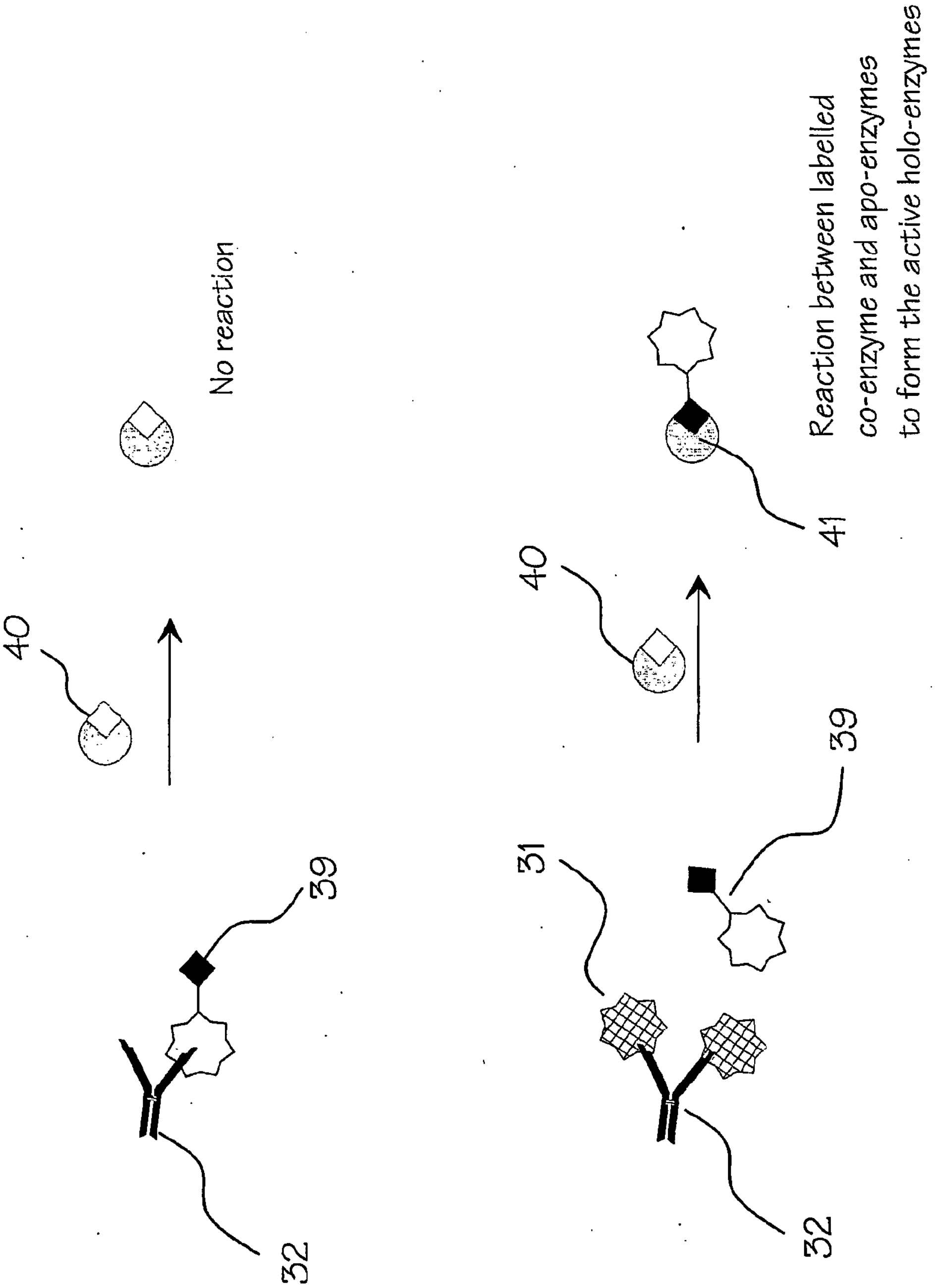
32
39
40
No reaction
31
32
39
40
41
Reaction between labelled
co-enzyme and apo-enzymes
to form the active holo-enzymes

Fig. 19

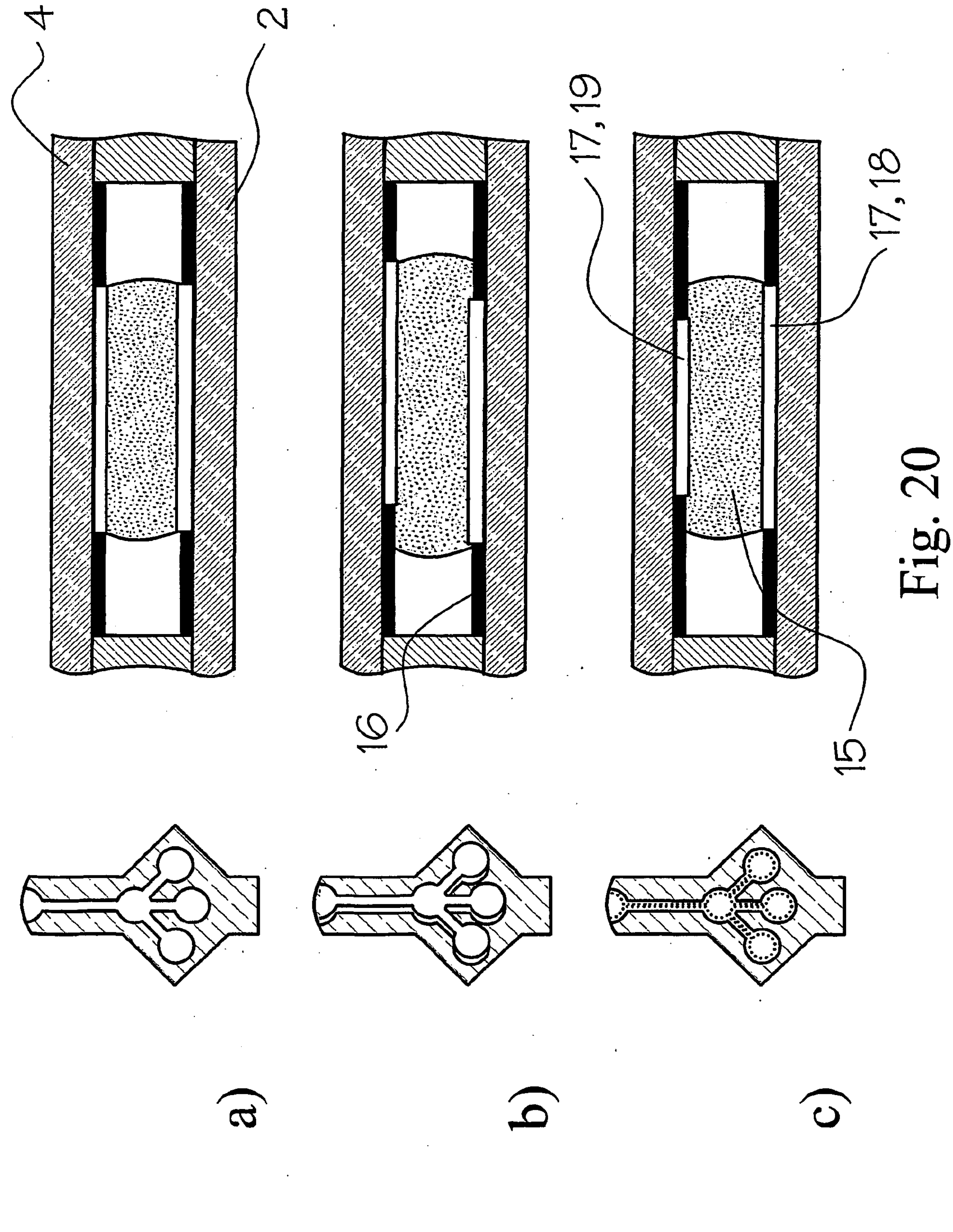


Fig. 20

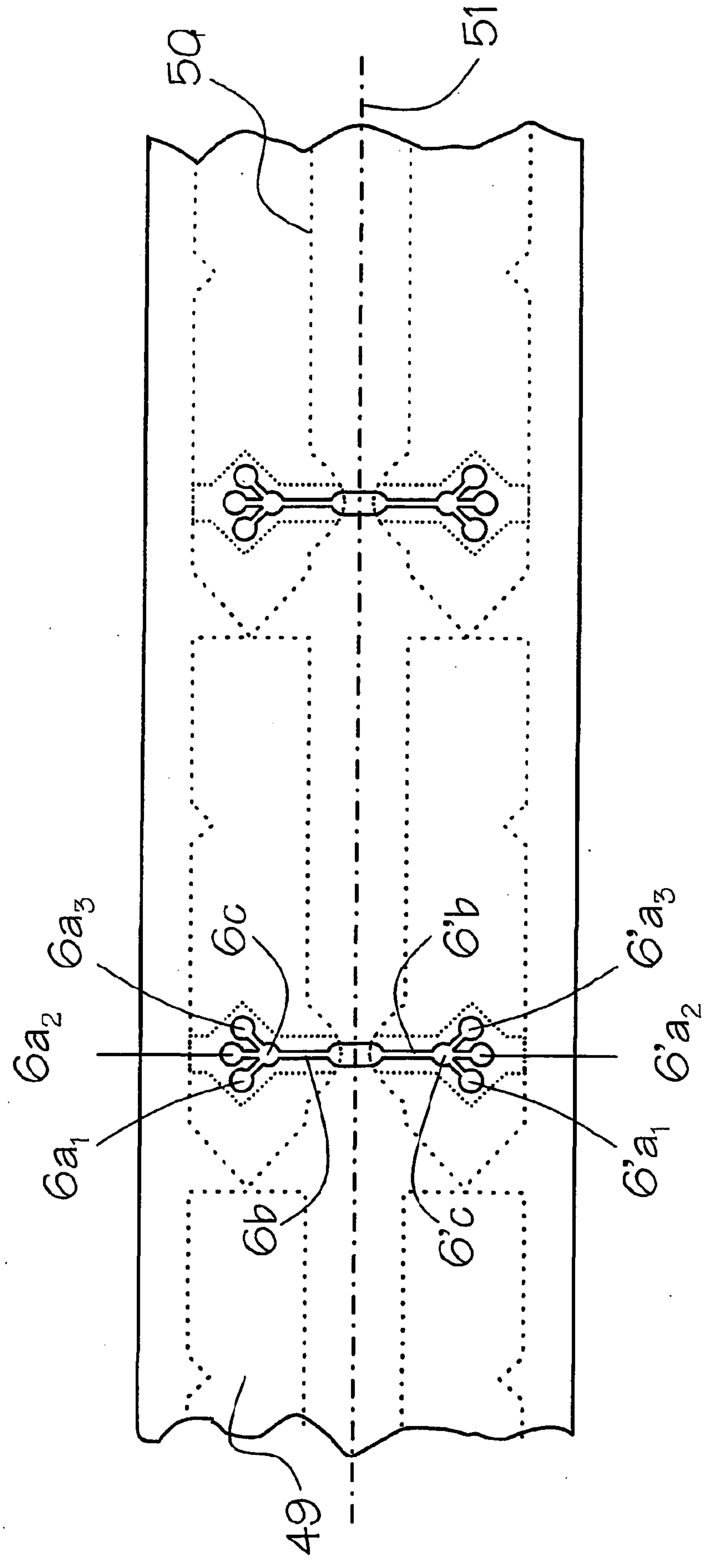


Fig. 21 a

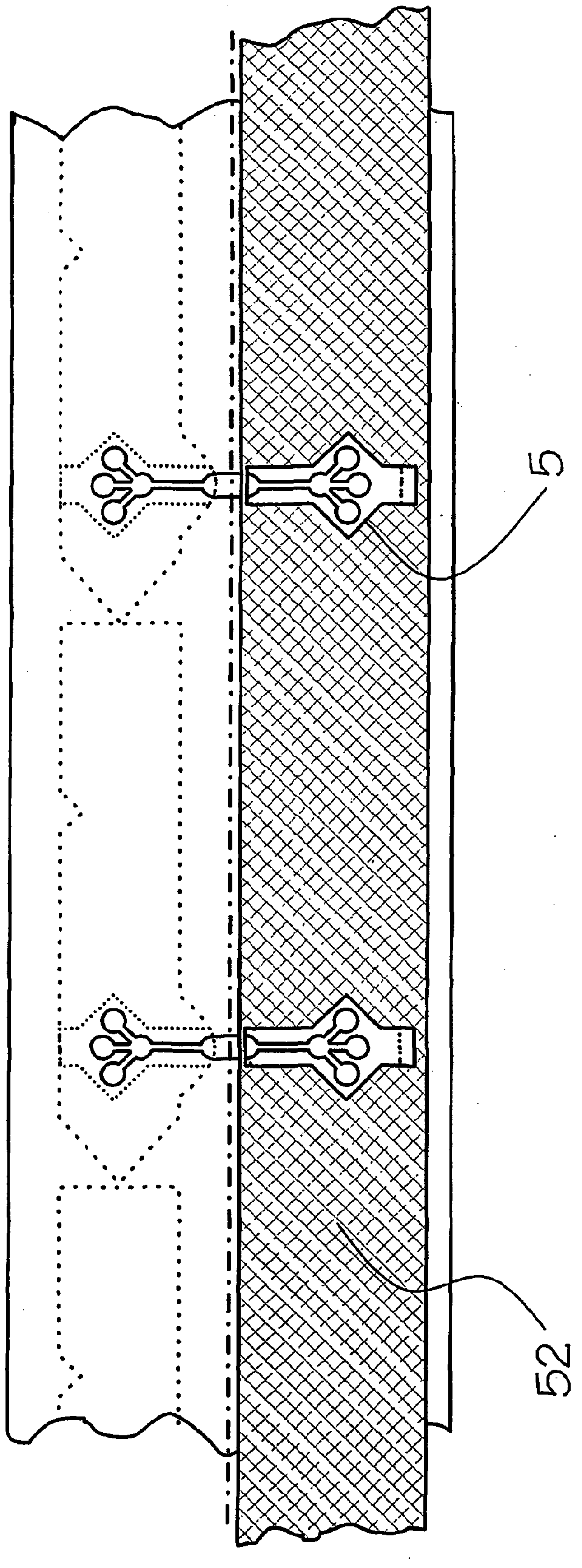


Fig. 21 b

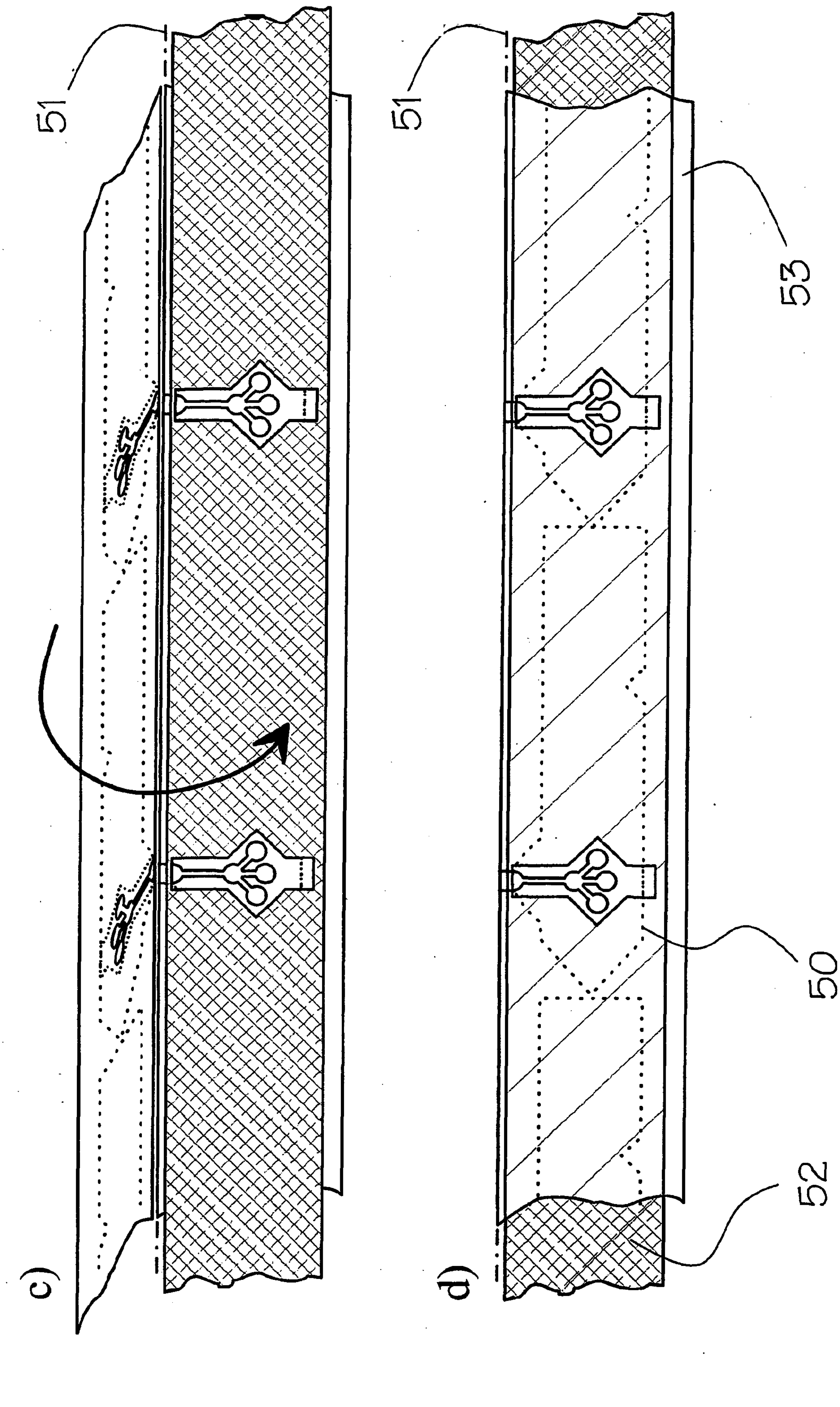


Fig. 21

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4213893 A
- US 4708933 A
- EP 163393 A
- WO 2002086472 A
- WO 2005098431 A
- EP 1574858 A1
- WO 2006015615 A
- US 6165739 A
- WO 02076878 A


### Non-patent literature cited in the description


- **RJ Tyhach.** Adaptation of Prostatic-Group-Label Homogeneous Immunoassay to reagent- strip format. *Clinical Chemistry,* 1981, vol. 27, 1499-1504
- The rate of colour generation is thus a function of the DNP-caproate concentration. *The Journal of Clinical Investigation,* 2005, vol. 115, 86-93
- **J.T. Kunjappu.** The Emergence of Polyacrylates in Ink Chemistry. Ink World, February 1999, 40
- **R. Golden.** *J. Coatings Technol.,* 1997, vol. 69, 83